# FASCICULE DE BREVET EUROPEEN

(11) **EP 2 534 241 B1**
(45) Date de publication et mention de la délivrance du brevet: **05.10.2016**
(21) Numéro de dépôt: 11708570.4
(22) Date de dépôt: 11.02.2011
(51) Int. Cl.: C12N 1/12, B09C 1/10, C02F 3/34, C07H 21/00, C22B 3/18, C22B 60/00, G21F 9/18

(54) **ALGUE RADIORESISTANTE DU GENRE COCCOMYXA**
RADIORESISTENTE ALGE VOM GENUS COCCOMYXA
RADIORESISTANT ALGA OF THE GENUS COCCOMYXA

(30) Priorité: 12.02.2010 FR 1000578
(43) Date de publication de la demande: 19.12.2012
(73) Titulaire: Commissariat à l'Energie Atomique, 75015 Paris (FR); Institut Max Von Laue - Paul Langevin, 38042 Grenoble Cedex 9 (FR); Centre National de la Recherche Scientifique, 75016 Paris (FR); MUSEUM NATIONAL D'HISTOIRE NATURELLE, 75005 Paris (FR)
(72) Inventeur: RIVASSEAU, Corinne, F-38210 Cras (FR); FARHI, Emmanuel, F-38210 Cras (FR); COUTE, Alain, F-91280 Saint-Pierre du Perray (FR); ATTEIA, Ariane, F-13009 Marseille (FR)
(74) Mandataire: Gevers & Orès
(86) Numéro de dépôt international: PCT/IB2011/050589
(87) Numéro de publication internationale: WO 2011/098979

(56) Documents cités:
- FAHRI E ET AL: "Spectoroscopic investigation of ionizing-radiation tolerance of a Chlorophyceae green micro-alga", JOURNAL OF PHYSICS: CONDENSED MATTER., vol. 20, 19 février 2008 (2008-02-19), pages 104216-104222, XP002599772, INSTITUTE OF PHYSICS PUBLISHING, BRISTOL ISSN: 0953-8984, DOI: 10.1088/0953-8984/20/10/104216
- RIVASSEAU C ET AL: "Resistance to irradiation of micro-algae growing in the storage pools of a nuclear reactor investigated by NMR and neutron spectroscopies", SPECTROSCOPY - FROM MOLECULE TO TISSUE: XIII EUROPEAN CONFERENCE ON THE SPECTROSCOPY OF BIOLOGICAL MOLECULES, PALERMO, ITALY, AUGUST 28-SEPTEMBER 2, 2009, PART 2 OF 2 2010 IOS PRESS NLD LNKD- DOI:10.3233/SPE-2010-0459, vol. 24, no. 3-4, 2 juillet 2010 (2010-07-02), pages 381-385, XP009138179, ISSN: 0712-4813
- GUSCHINA I A ET AL: "Lead and copper effects on lipid metabolism in cultured lichen photobionts with different phosphorus status", PHYTOCHEMISTRY, PERGAMON PRESS, GB LNKD- DOI:10.1016/J.PHYTOCHEM.2006.01.023, vol. 67, no. 16, 1 août 2006 (2006-08-01), pages 1731-1739, XP025231016, ISSN: 0031-9422 [extrait le 2006-08-01]
- ZOLLER STEFAN ET AL: "Slow algae, fast fungi: Exceptionally high nucleotide substitution rate differences between lichenized fungi Omphalina and their symbiotic green algae Coccomyxa.", MOLECULAR PHYLOGENETICS AND EVOLUTION, vol. 29, no. 3, décembre 2003 (2003-12), pages 629-640, XP002599256, ISSN: 1055-7903
- DATABASE EMBL [en ligne] 02 Décembre 2006 'Coccomyxa glaronensis 18S rRNA gene, strain CCALA 306' Retrieved from EBI, accession no. EM_STD:AM167525 Database accession no. AM167525

### SEQUENCE LISTING

<110> COMMISSARIAT A L'ENERGIE ATOMIQUE
   INSTITUT MAX VON LAUE - PAUL LANGEVIN
   CENTRE NATIONAL DE LA RECHERCHE SCIENTIFIQUE
   MUSEUM NATIONAL D'HISTOIRE NATURELLE
   RIVASSEAU, Corinne
   FARHI, Emmanuel
   COUTE, Alain
   ATTEIA, Ariane
<120> Nouvelle algue radiorésistante du genre Coccomyxa
<130> MJP/EM/11/F263-354WO
<150> FR 10 00578
   <151> 2010-02-12
<160> 13
<170> PatentIn version 3.5
<210> 1
   <211> 4065
   <212> DNA
   <213> Coccomyxa actinabiotis
<220>
   <221> rRNA
   <222> (1)..(4065)
<220>
   <221> misc_feature
   <222> (3043)..(3043)
   <223> n = a, c, g, or t
<220>
   <221> misc_feature
   <222> (3195)..(3195)
   <223> n = a, c, g, or t
<220>
   <221> misc_feature
   <222> (4013)..(4016)
   <223> n = a, c, g, or t
<400> 1
   cgacaatctg gttgatcctg ccagtagtca tatgcttgtc tcaaagatta agccatgcat      60
   gtctaagtat aaactgcttt atactgtgaa actgcgaatg gctcattaaa tcagttatag     120
   tttatttgat ggtaccttgc tactcggata accgtagtaa ttctagagct aatacgtgcg     180
   taaatcccga cttctggaag ggacgtattt attagataaa aggccgaccg gactctgtcc     240
   gactcgcggt gaatcatgat aactccacgg atcgcatggc ctcgagccgg cgatgtttca     300
   ttcaaatttc tgccctatca actttcgacg gtaaggtatt ggcttaccgt ggtggtaacg     360
   ggtgacggag gattagggtt cgattccgga gagggagcct gagaaacggc taccacatcc     420
   aaggaaggca gcaggcgcgc aaattaccca atcttgacac aaggaggtag tgacaataaa       480
   taacaatacc ggggtttttc aactctggta attggaatga gtacaatcta aaccccttaa       540
   cgaggatcaa ttggagggca agtctggtgc cagcagccgc ggtaattcca gctccaatag      600
   cgtatattta agttgttgca gttaaaaagc tcgtagttgg atttcgggcg ggctcggccg      660
   gtccgccgtt cggtgtgcac tgaccgagcc cgtcttgttg ccggggacgg gctcctgggc      720
   ttaactgtcc gggactcgga gtcggcgagg ttactttgag taaattagag tgttcaaagc      780
   aggcctacgc tctgaataca ttagcatgga ataacacgaa taggactctg gcctatcttg      840
   ttggtctgtg ggaccggagt aatgattaag agggacagtc gggggcattc gtatttcatt      900
   gtcagaggtg aaattcttgg atttatgaaa gacgaactac tgcgaaagca tttgccaagg      960
   atgttttcat taatcaagaa cgaaagttgg gggctcgaag acgattagat accgtcctag     1020
   tctcaaccat aaacgatgcc gactagggat tggcgggcgt tcttttgatg acctcgccag     1080
   caccttatga gaaatcaaag tttttgggtt ccggggggag tatggtcgca aggctgaaac     1140
   ttaaaggaat tgacggaagg gcaccaccag gcgtggaatg ttcatggctt tgcgcctcag     1200
   agggatgctt taacgagtat ccctagtgcc tggtttcagg accaggcgca acaccgtcca     1260
   attgcgggga ctccctaaag ctcgtgcaca ccaagcggtg aggggaaacc ccatcgtggc     1320
   caggccaatc acctgggtaa ggtaacagtg gcgcgggatg aaggcgcagt aatgtgcctg     1380
   aaatgggtga tccgcagcca agtcctacag agcccggcct gcccgggttc cacggatgca     1440
   gctcacagac taaatggcgg tgggtgtgat gcaatcattt aggaaacaca cgttcaggaa     1500
   ttcaagacaa agaattgtgg attgtgaaca tgtgaatttg tcaaataaat gattgtaacc     1560
   atgcttaaga tatagtcgga ccacctcgaa agaggcaccg acgagaggat ccgatcaata     1620
   gaccggggag agctcgtcgg gggtggcggt agtagttgct ggagcaatcc tgtcaatggc     1680
   tgaaacgaag tttcttgcct cgccatccgg agtgttcggg agcctgcggc ttaatttgac     1740
   tcaacacggg aaaacttacc aggtccagac atagtgagga ttgacagatt gagagctctt     1800
   tcttgattct atgggtggtg gtgcatggcc gttcttagtt ggtgggttgc cttgtcaggt     1860
   tgattccggt aacgaacgag acctcagcct gctaactagt cacggttggt ttttccagcc      1920
   ggcggacttc ttagagggac tattggcgac tagccaatgg aagtgtgagg caataacagg     1980
   tctgtgatgc ccttagatgt tctgggccgc acgcgcgcta cactgatgca atcaacgagc     2040
   ctagccttgg ccgagaggtc cgggtaatct ttgaaactgc atcgtgatgg ggatagatta     2100
   ttgcaattat taatcttcaa cgaggaatgc ctagtaagcg cgagtcatca gctcgcgttg     2160
   attacgtccc tgccctttgt acacaccgcc cgtcgctcct accgattggg tgtgctggtg     2220
   aagcgttcgg attggcggct tcaggcggtt cgccgcccga tgcagccgag aagttcgtta     2280
   aaccctccca cctagaggaa ggagaagtcg taacaaggtt tccgtagaag aatactgttt     2340
   gttaacaccg tgcggaacac agctcttcct tgtgtgctag tggggccctg gagccctgcg     2400
   acactgtcaa attgcctgga actcccgctt ggatgtagtc cggtgccacc gcttccgatt     2460
   ggaaacagtc gggagcaccg tcgggaaagc cgacgggtac ggtaacagag cgccggatga 2520
   gggacgatgg gcagccaagt cctaagtgcg caagcatatg gatgcagttc acagactaaa 2580
   tggcagtggg ttcctcagcg ccgatcagaa gcagaggagc ttaagatata gtcggaccgc 2640
   cccgtgaggg gagccggcag gaggatcccg gttcagcaac cggcgagaga gcctgtcggg 2700
   agcggcctaa tagttggcgt ctgtcaacgg cacacgtggg cctgccgctc ggagtagtcg 2760
   aggtgaacct gcggaaggat cattgcatcg atcaaacaca aaccgcgaac cacgtatccc 2820
   cgctgggcgg ctcctctttt gttatatcga aggaaggccg aacagtgcgc ttgtccctga 2880
   cccgggtcct cggctttcga ggctcggcag gtctggcggg ccggggagac ctctctccga 2940
   tcctgacccg atcggatgac gtcttctgtc ggtcggcaaa acccccaatc acaaccgaac 3000
   gtaacgaagc ctgaagcaat cggcgtgctg tcatcagccg tcncgaaacc aatgacaact 3060
   ctcaacaatg gatgtcttgg ctcccgtatc gatgaagaac gcagcgaaat gcgatacgta 3120
   gtgtgaattg cagataatgt gaatcatcga atctttgaac gcaaattgca ctcgaggctc 3180
   cggccaagag tatgnctgcc tcagcgtcgg ctcaccccct cgcctcccct ttgtctccta 3240
   ccgaacaagg aaggatggcg gacgtggcgg tcccggtgaa ctttccttcg aaggacaacc 3300
   gggtccgctg aagaccagag gctcgagcaa ggacccattc gggacaaaca gtctggtagg 3360
   agcgtcgtct cagacctcat gcccgctgtc ggcccgggac tttgttggag gccggaaggc 3420
   gctcctcggt tatggtaacc ggagctcttc atgaaatatt cgacctgagt tcaggcaagg 3480
   gcacccgctg aacttaagca tatcaataag cggaggaaaa gaaactaacc aggattcccc 3540
   tagtagcggc gagcgaaccg ggaagagccc aacttgaaaa tctccgtctg ccaggcggcg 3600
   aatgtagtct agagaggcgt cctctgtggc gggtcgtgcc caagtccctt ggaagggggc 3660
   gtccgagagg gtgagaaccc cgtcggccac ggcctcagcc actccacgag gcgctttcgc 3720
   agagtcgggt tgtttgggaa tgcagcccaa agcaggtggt aagtcccatc taaggctaaa 3780
   tactgacggg agaccgatag cgaacaagta ccgcgaggga aagatgaaaa gaactttgaa 3840
   aagagagtta aaaagtgcct gaaattgttg agggggaagg gattggaggt cgcgggtgcg 3900
   cccaggcaaa cgccttttac gctaaaaggc tgaatgtgct gggcgctggt cagcgtcggt 3960
   tagccgggcg ggacaacagc ggtctttgat cggccgcctc tgtcgcccgg ctnnnngagg 4020
   aacgaagggt gctctggcga ttcgtcgacc tgcgctctca ggatg 4065
<210> 2
   <211> 25
   <212> DNA
   <213> Artificial
<220>
   <223> Amorce PCR
<400> 2
   tcgacaatct ggttgatcct gccag 25
<210> 3
   <211> 26
   <212> DNA
   <213> Artificial
<220>
   <223> Amorce PCR
<400> 3
   ctccttggtc cgtgtttcaa gacggg 26
<210> 4
   <211> 2354
   <212> DNA
   <213> Coccomyxa actinabiotis
<220>
   <221> rRNA
   <222> (1)..(2354)
<400> 4
   cgacaatctg gttgatcctg ccagtagtca tatgcttgtc tcaaagatta agccatgcat      60
   gtctaagtat aaactgcttt atactgtgaa actgcgaatg gctcattaaa tcagttatag     120
   tttatttgat ggtaccttgc tactcggata accgtagtaa ttctagagct aatacgtgcg     180
   taaatcccga cttctggaag ggacgtattt attagataaa aggccgaccg gactctgtcc     240
   gactcgcggt gaatcatgat aactccacgg atcgcatggc ctcgagccgg cgatgtttca     300
   ttcaaatttc tgccctatca actttcgacg gtaaggtatt ggcttaccgt ggtggtaacg     360
   ggtgacggag gattagggtt cgattccgga gagggagcct gagaaacggc taccacatcc     420
   aaggaaggca gcaggcgcgc aaattaccca atcttgacac aaggaggtag tgacaataaa     480
   taacaatacc ggggtttttc aactctggta attggaatga gtacaatcta aaccccttaa     540
   cgaggatcaa ttggagggca agtctggtgc cagcagccgc ggtaattcca gctccaatag     600
   cgtatattta agttgttgca gttaaaaagc tcgtagttgg atttcgggcg ggctcggccg     660
   gtccgccgtt cggtgtgcac tgaccgagcc cgtcttgttg ccggggacgg gctcctgggc     720
   ttaactgtcc gggactcgga gtcggcgagg ttactttgag taaattagag tgttcaaagc     780
   aggcctacgc tctgaataca ttagcatgga ataacacgaa taggactctg gcctatcttg     840
   ttggtctgtg ggaccggagt aatgattaag agggacagtc gggggcattc gtatttcatt     900
   gtcagaggtg aaattcttgg atttatgaaa gacgaactac tgcgaaagca tttgccaagg     960
   atgttttcat taatcaagaa cgaaagttgg gggctcgaag acgattagat accgtcctag    1020
   tctcaaccat aaacgatgcc gactagggat tggcgggcgt tcttttgatg acctcgccag    1080
   caccttatga gaaatcaaag tttttgggtt ccggggggag tatggtcgca aggctgaaac    1140
   ttaaaggaat tgacggaagg gcaccaccag gcgtggaatg ttcatggctt tgcgcctcag    1200
   agggatgctt taacgagtat ccctagtgcc tggtttcagg accaggcgca acaccgtcca 1260
   attgcgggga ctccctaaag ctcgtgcaca ccaagcggtg aggggaaacc ccatcgtggc 1320
   caggccaatc acctgggtaa ggtaacagtg gcgcgggatg aaggcgcagt aatgtgcctg 1380
   aaatgggtga tccgcagcca agtcctacag agcccggcct gcccgggttc cacggatgca 1440
   gctcacagac taaatggcgg tgggtgtgat gcaatcattt aggaaacaca cgttcaggaa 1500
   ttcaagacaa agaattgtgg attgtgaaca tgtgaatttg tcaaataaat gattgtaacc 1560
   atgcttaaga tatagtcgga ccacctcgaa agaggcaccg acgagaggat ccgatcaata 1620
   gaccggggag agctcgtcgg gggtggcggt agtagttgct ggagcaatcc tgtcaatggc 1680
   tgaaacgaag tttcttgcct cgccatccgg agtgttcggg agcctgcggc ttaatttgac 1740
   tcaacacggg aaaacttacc aggtccagac atagtgagga ttgacagatt gagagctctt 1800
   tcttgattct atgggtggtg gtgcatggcc gttcttagtt ggtgggttgc cttgtcaggt 1860
   tgattccggt aacgaacgag acctcagcct gctaactagt cacggttggt ttttccagcc 1920
   ggcggacttc ttagagggac tattggcgac tagccaatgg aagtgtgagg caataacagg 1980
   tctgtgatgc ccttagatgt tctgggccgc acgcgcgcta cactgatgca atcaacgagc 2040
   ctagccttgg ccgagaggtc cgggtaatct ttgaaactgc atcgtgatgg ggatagatta 2100
   ttgcaattat taatcttcaa cgaggaatgc ctagtaagcg cgagtcatca gctcgcgttg 2160
   attacgtccc tgccctttgt acacaccgcc cgtcgctcct accgattggg tgtgctggtg 2220
   aagcgttcgg attggcggct tcaggcggtt cgccgcccga tgcagccgag aagttcgtta 2280
   aaccctccca cctagaggaa ggagaagtcg taacaaggtt tccgtagaag aatactgttt 2340
   gttaacaccg tgcg 2354
<210> 5
   <211> 1783
   <212> DNA
   <213> Coccomyxa peltigerae souche SAG 216-5
<220>
   <221> rRNA
   <222> (1)..(1783)
<400> 5
   tcgacaatct ggttgatcct gccagtagtc atatgcttgt ctcaaagatt aagccatgca      60
   tgtctaagta taaactgctt tatactgtga aactgcgagt ggctcgttaa atcagttata     120
   gtttatttga tggtacctta ctactcggat aaccgtagta attctagagc taatacgtgc     180
   ggaaatcccg acttccggaa gggacgtatt tattagataa aaggccgacc gggcttgccc     240
   gaaacgcggt gaatcatgat aactccacga atcgcatggc ctcagtgccg gcgatgtttc     300
   attcaaattt ctgccctatc aactttcgac ggtaaggtat tggcttaccg tggtggtaac     360
   gggtgacgga ggattggggt tcgattccgg agagggagcc tgagaaacgg caaccacatc     420
   caaggaaggc agcaggcgcg caaattaccc aatcttgaca caaggaggta gtgacaatga     480
   ataacaatac cggggttttt caactctggt aattggaatg agtacaatct aaacccctta     540
   acgaggatca attggagggc aagtctggtg ccagcagccg cggtaattcc agctccaata     600
   gcgtatattt aagttgttgc agttaaaaag ctcgtagttg gatttcgggc gggcccggcc     660
   ggtccgcctt cgggtgtgca ctgaccgggc ccgtcatgtt gccggggacg ggctcctggg     720
   cttcactgtc cgggactcgg agtcggcgag gttactttga gtaaattaga gtgttcaaag     780
   caggcctacg ctctgaatac attagcatgg aataacacga taggactctg gcctatcttg     840
   ttggtctgtg ggaccggagt aatgattaag agggacagtc gggggcattc gtatttcatt     900
   gtcagaggtg aaattcttgg atttatgaaa gacgaactac tgcgaaagca tttgccaagg     960
   atgttttcat taatcaagaa cgaaagttgg gggctcgaag acgattagat accgtcctag    1020
   tctcaaccat aaacgatgcc gactagggat tggcgggcgt tcttttgatg accccgccag    1080
   caccttatga gaaatcaaag tttttgggtt ccggggggag tatggtcgca aggctgaaac    1140
   ttaaaggaat tgacggaagg gcaccaccag gcgtggagcc tgcggcttaa tttgactcaa    1200
   cacgggaaaa cttaccaggt ccagacatag tgaggattga cagattgaga gctctttctt    1260
   gattctatgg gtggtggtgc atggccgttc ttagttggtg ggttgccttg tcaggttgat    1320
   tccggtaacg aacgagacct cagcctgcta actagtcacg attggttctt ccagtcggcc    1380
   ggcttcttag agggactatt ggcgactagc caatggaagt gtgaggcaat aacaggtctg    1440
   tgatgccctt agatgttctg ggccgcacgc gcgctacact gatgcagtca acgagcctag    1500
   ccttggccga caggtccggg taatctttga aactgcatcg tgatggggat agatgattgc    1560
   aattattcat cttcaacgag gaatgcctag taagcgcgag tcatcagctc gcgttgatta    1620
   cgtccctgcc ctttgtacac accgcccgtc gctcctaccg attgggtgtg ctggtgaagc    1680
   gttcggattg gcggcagtgc gcggttcgcc gctcgctgca gccgagaagt tcgttaaacc    1740
   ctcccaccta gaggaaggag aagtcgtaac aaggtttccg tag                       1783
<210> 6
   <211> 1783
   <212> DNA
   <213> Coccomyxa chodatii souche SAG 216-2
<220>
   <221> rRNA
   <222> (1)..(1783)
<400> 6
   tcgacaatct ggttgatcct gccagtagtc atatgcttgt ctcaaagatt aagccatgca 60
   tgtctaagta taaactgctt tatactgtga aactgcgaat ggctcattaa atcagttata 120
   gtttatttga tggtacctta ctactcggat aaccgtagta attctagagc taatacgtgc 180
   ggaaatcccg acttctggaa gggacgtatt tattagataa aaggccgacc gggcttgccc      240
   gaaacgcggt gaatcatgat aactccacga atcgcatggc ctcagtgccg gcgatgtttc      300
   attcaaattt ctgccctatc aactttcgac ggtaaggtat tggcttaccg tggtggtaac      360
   gggtgacgga ggattagggt tcgattccgg agagggagcc agagaaacgg ctaccacatc      420
   caaggaaggc agcaggcgcg caaattaccc aatcttgaca caaggaggta gtgacaataa      480
   ataacaatac cggggttttt caactctggt aattggaatg agtacaatct aaacccctta      540
   acgaggatca attggagggc aagtctggtg ccagcagccg cggtaattcc agctccaata      600
   gcgtatattt aagttgttgc agttaaaaag ctcgtagttg gatctcgggc gggcccggcc      660
   ggtccgcctt cgggtgtgca ctgaccgggc ccgtcttgtt gccggggacg ggctcctggg      720
   cttcactgtc cgggactcgg agtcggcgag gttactttga gtaaattaga gtgttcaaag      780
   caggcctacg ctctgaatac attagcatgg aataacacga taggactctg gcctatcttg      840
   ttggtctgtg ggaccggagt gatgattaag agggacagtc gggggcattc gtatttcatt      900
   gtcagaggtg aaattcttgg atttatgaaa gacgaactac tgcgaaagca tttgccaagg      960
   atgttttcat taatcaagaa cgaaagttgg gggctcgaag acgattagat accgtcctag     1020
   tctcaaccat aaacgatgcc gactagggat tggcgggcgt tcttttgatg accccgccag     1080
   caccttatga gaaatcaaag tttttgggtt ccggggggag tatggtcgca aggctgaaac     1140
   ttaaaggaat tgacggaagg gcaccaccag gcgtggagcc tgcggcttaa tttgactcaa     1200
   cacgggaaaa cttaccaggt ccagacatag tgaggattga cagattgaga gctctttctt     1260
   gattctatgg gtggtggtgc atggccgttc ttagttggtg ggttgccttg tcaggttgat     1320
   tccggtaacg aacgagacct cagcctgcta actagtcacg attggttctt ccagtcggcc     1380
   gacttcttag agggactatt ggcgactagc caatggaagt gtgaggcaat aacaggtctg     1440
   tgatgccctt agatgttctg ggccgcacgc gcgctacact gatgcaatca acgagcctag     1500
   ccttggccga caggtccggg taatctttga aactgcatcg tgatggggat agatgattgc     1560
   aattattcat cttcaacgag gaatgcctag taagcgcgag tcatcagctc gcgttgatta     1620
   cgtccctgcc ctttgtacac accgcccgtc gctcctaccg attgggtgtg ctggtgaagc     1680
   gttcggattg gcggcagtgc gcggttcgcc gctcgctgca gccgagaagt tcgttaaacc     1740
   ctcccaccta gaggaaggag aagtcgtaac aaggtttccg tag                       1783
<210> 7
   <211> 1778
   <212> DNA
   <213> Coccomyxa sp. Flensburg fjord 2
<220>
   <221> rRNA
   <222> (1)..(1778)
<400> 7
   acctggttga tcctgccagt agtcatatgc ttgtctcaaa gattaagcca tgcatgtcta 60
   agtataaact gatttatact gtgaaactgc gaatggctca ttaaatcagt tatagtttat 120
   ttgatggtac cttgctactc ggataaccgt agtaattcta gagctaatac gtgcggaaat 180
   cccgactcct ggaagggacg tatttattag ataaaaggcc gaccggactc cgtccgactc 240
   gcggtgaatc atgataactc cacgaatcgc atggcccagc gccggcgatg tttcattcaa 300
   atttctgccc tatcaacttt cgacggtaag gtattggctt accgtggtgg taacgggtga 360
   cggaggatta gggttcgatt ccggagaggg agcctgagaa acggctacca catccaagga 420
   aggcagcagg cgcgcaaatt acccaatctt gacacaagga ggtagtgaca ataaataaca 480
   ataccggggt tattcaactc tggtaattgg aatgagtaca atctaaaccc cttaatgagg 540
   atcaattgga gggcaagtct ggtgccagca gccccggtaa ttccagctcc aatagcgtat 600
   atttaagttg ttgcagttaa aaagctcgta gttggatttc gggcgggccc ggccggtccg 660
   cctcacggtg tgcactgacc gggcccgtcc tgctgccggg gacgggctcc tgggcttcac 720
   tgtccgggac tcggagtcgg cgaggttact ttgagtaaat tagagtgttc aaagcaggca 780
   tccgccttga atacgttagc atggaataac acgataggac tctggcctat cttgttggtc 840
   tgtgggaccg gagtaatgat taagagggac agtcgggggc attcgtattt cattgtcaga 900
   ggtgaaattc ttggatttat gaaagacgaa ctactgcgaa agcatttgcc aaggatgttt 960
   tcattaatca agaacgaaag ttgggggctc gaagacgatt agataccgtc ctagtctcaa 1020
   ccataaacga tgccgactag ggattggcgg gcgttctatt gatgaccccg ccagcacctt 1080
   atgagaaatc aaagtttttg ggttccgggg ggagtatggt cgcaaggctg aaacttaaag 1140
   gaattgacgg aagggcacca ccaggcgtgg agcctgccgc ttaatttgac tcaacacggg 1200
   aaaacttacc aggtccagac atagtgagga ttgacagatt gaaagctctt tcttgattct 1260
   atgggtggtg gtgcatggcc gttcttagtt ggtgggttgc cttgtcaggt tgattccggt 1320
   aacgaacgag acctcagcct gctaactagt cacggctgga ttctccagcc ggcggacttc 1380
   ttagagggac tattggcgac tagccaatgg aagtgtgagg caataacagg tctgtgatgc 1440
   ccttagatgt tctgggccgc acgcgcgcta cactgatgcg atcaacgagc ctagccttgg 1500
   ccgacaggtc cgggtaatct tgcaaaccgc atcgtgatgg ggatagatta ttgcaattat 1560
   taatcttcaa cgaggaatgc ctagtaggcg cgagtcatca gctcgcgtcg attacgtccc 1620
   tgccctttgt acacaccgcc cgtcgctcct accgattggg tgtgctggtg aagcgttcgg 1680
   attggcggcc tccggcggtt cgccgctggg agcagccgag aagttcgtta aaccctccca 1740
   cctagaggaa ggagaagtcg taacaaggtt tccgtagg 1778
<210> 8
   <211> 1797
   <212> DNA
   <213> Coccomyxa glaronensis souche CCALA 306
<400> 8
   aacctggttg atcctgccag tagtcatatg cttgtctcaa agattaagcc atgcatgtct      60
   aagtataaac tgctttatac tgtgaaactg cgaatggctc attaaatcag ttatagttta     120
   tttgatggta ccttgctact cggataaccg tagtaattct agagctaata cgtgcggaaa     180
   tcccgactcc tggaagggac gtatttatta gataaaaggc cgaccggact cgtccgactc     240
   gcggtgaatc atgataactc cacgaatcgc atggcccagc gccggcgatg tttcattcaa     300
   atttctgccc tatcaacttt cgacggtaag gtattggctt accgtggtgg taacgggtga     360
   cggaggatta gggttcgatt ccggagaggg agcctgagaa acggctacca catccaagga     420
   aggcagcagg cgcgcaaatt acccaatctt gacacaagga ggtagtgaca ataaataaca     480
   ataccggggt ttttcaactc tggtaattgg aatgagtaca atctaaaccc cttaacgagg      540
   atcaattgga gggcaagtct ggtgccagca gccgcggtaa ttccagctcc aatagcgtat     600
   atttaagttg ttgcagttaa aaagctcgta gttggatttc gggcgggccc ggccggtccg     660
   cctcacggtg tgcactgacc gggcccgtcc tgctgccggg gacgggctcc tgggcttcac     720
   tgtccgggac tcggagtcgg cgaggttact ttgagtaaat tagagtgttc aaagcaggca     780
   tccgccttga atacgttagc atggaataac acgataggac tctggcctat cttgttggtc     840
   tgtgggaccg gagtaatgat taagagggac agtcgggggc attcgtattt cattgtcaga     900
   ggtgaaattc ttggatttat gaaagacgaa ctactgcgaa agcatttgcc aaggatgttt     960
   tcattaatca agaacgaaag ttgggggctc gaagacgatt agataccgtc ctagtctcaa    1020
   ccataaacga tgccgactag ggattggcgg gcgttctatt gatgaccccg ccagcacctt    1080
   atgagaaatc aaagtttttg ggttccgggg ggagtatggt cgcaaggctg aaacttaaag    1140
   gaattgacgg aagggcacca ccaggcgtgg agcctgcggc ttaatttgac tcaacacggg    1200
   aaaacttacc aggtccagac atagtgagga ttgacagatt gagagctctt tcttgattct    1260
   atgggtggtg gtgcatggcc gttcttagtt ggtgggttgc cttgtcaggt tgattccggt    1320
   aacgaacgag acctcagcct gctaactagt cacggctgcc ccggcagccg gcggacttct    1380
   tagagggact attggcgact agccaatgga agtgtgaggc aataacaggt ctgtgatgcc    1440
   cttagatgtt ctgggccgca cgcgcgctac actgatgcga tcaacgagcc tagccttggc    1500
   cgacaggtcc gggtaatctt gcaaaccgca tcgtgatggg gatagattat tgcaattatt    1560
   aatcttcaac gaggaatgcc tagtaggcgc gagtcatcag ctcgcgtcga ttacgtccct    1620
   gccctttgta cacaccgccc gtcgctccta ccgattgggt gtgctggtga agcgttcgga    1680
   ttggcggcct ccggcggttc gccgctggga gcagccgaga agttcgttaa accctcccac    1740
   ctagaggaag gagaagtcgt aacaaggttt ccgtaggtga acctgcrgaa ggatcaa       1797
<210> 9
   <211> 1797
   <212> DNA
   <213> Coccomyxa sp. souche CPCC 508
<220>
   <221> rRNA
   <222> (1)..(1797)
<400> 9
   wacctggttg atcctgccag tagtcatatg cttgtctcaa agattaagcc atgcatgtct      60
   aagtataaac tgctttatac tgtgaaactg cgaatggctc attaaatcag ttatagttta     120
   tttgatggta ccttgctact cggataaccg tagtaattct agagctaata cgtgcggaaa     180
   tcccgactcc tggaagggac gtatttatta gataaaaggc cgaccggact ctgtccgact     240
   cgcggtgaat catgataact ccacgaatcg catggcccag cgccggcgat gtttcattca     300
   aatttctgcc ctatcaactt tcgacggtaa ggtattggct taccgtggtg gtaacgggtg     360
   acggaggatt agggttcgat tccggagagg gagcctgaga aacggctacc acatccaagg     420
   aaggcagcag gcgcgcaaat tacccaatct tgacacaagg aggtagtgac aataaataac     480
   aataccgggg ttattcaact ctggtaattg gaatgagtac aatctaaacc ccttaacgag     540
   gatcaattgg agggcaagtc tggtgccagc agccgcggta attccagctc caatagcgta     600
   tatttaagtt gttgcagtta aaaagctcgt agttggattt cgggcgggcc cggccggtcc     660
   gcctcacggt gtgcactgac cgggcccgtc ctgctgccgg ggacgggctc ctgggcttca     720
   ctgtccggga ctcggagtcg gcgaggttac tttgagtaaa ttagagtgtt caaagcaggc     780
   atccgccttg aatacgttag catggaataa cacgatagga ctctggccta tcttgttggt     840
   ctgtgggacc ggagtaatga ttaagaggga cagtcggggg cattcgtatt tcattgtcag     900
   aggtgaaatt cttggattta tgaaagacga actactgcga aagcatttgc caaggatgtt     960
   ttcattaatc aagaacgaaa gttgggggct cgaagacgat tagataccgt cctagtctca    1020
   accataaacg atgccgacta gggattggcg ggcgttctat tgatgacccc gccagcacct    1080
   tatgagaaat caaagttttt gggttccggg gggagtatgg tcgcaaggct gaaacttaaa    1140
   ggaattgacg gaagggcacc accaggcgtg gagcctgcgg cttaatttga ctcaacacgg    1200
   gaaaacttac caggtccaga catagtgagg attgacagat tgagagctct ttcttgattc    1260
   tatgggtggt ggtgcatggc cgttcttagt tggtgggttg ccttgtcagg ttgattccgg    1320
   taacgaacga gacctcagcc tgctaactag tcacggctgg attctccagc cggcggactt    1380
   cttagaggga ctattggcga ctagccaatg gaagtgtgag gcaataacag gtctgtgatg    1440
   cccttagatg ttctgggccg cacgcgcgct acactgatgc gatcaacgag cctagccttg    1500
   gccgacaggt ccgggtaatc ttgcaaaccg catcgtgatg gggatagatt attgcaatta    1560
   ttaatcttca acgaggaatg cctagtaggc gcgagtcatc agctcgcgtc gattacgtcc    1620
   ctgccctttg tacacaccgc ccgtcgctcc taccgattgg gtgtgctggt gaagcgttcg    1680
   gattggcggc ctccggcggt tcgccgctgg gagcagccga gaagttcgtt aaaccctccc    1740
   acctagagga aggagaagtc gtaacaaggt ttccgtaggt gaacctgcrg aaggatc       1797
<210> 10
   <211> 1558
   <212> DNA
   <213> Chlamydomonas sp. CCMP681
<220>
   <221> rRNA
   <222> (1)..(1558)
<400> 10
   gctcattaat cagttatagt ttatttgatg gtacctttac tcggataacc gtagtaattc      60
   tagagctaat acgtgcgtaa atcccgactt ctggaaggga cgtatttatt agataaaagg     120
   ccagccgggc ttgcccgacc ttaggcgaat catgataact tcacgaatcg catggccttg     180
   tgccggcgat gtttcattca aatttctgcc ctatcaactt tcgatggtag gatagaggcc     240
   taccatggtg gtaacgggtg acggaggatt agggttcgat tccggagagg gagcctgaga     300
   aacggctacc acatccaagg aaggcagcag gcgcgcaaat tacccaatcc cgacacgggg     360
   aggtagtgac aataaataac aataccgggc atctttgtct ggtaattgga atgagtacaa     420
   tgtaaatatc ttaacgagta tccattggag ggcaagtctg gtgccagcag ccgcggtaat     480
   tccagctcca atagcgtata tttaagttgt tgcagttaaa aagctcgtag ttggatttcg     540
   ggtgggttgc agtggtctgc cactggtatg tactgctgcg gctcaccttt ctgctgggga     600
   cgggctcctg ggcttaactg tctgggactc ggaatcagcg aagtgacctt gagcaaaagt     660
   gagtgttcaa agcaagccta cgctctgaaa catttagcat gggatcacac gataggactc     720
   tggcctatct tgttggtctg taggaccgga gtaatgatta agagggacag tcgggggcat     780
   tcgtatttca ttgtcagagg tgaaattctt ggatttatga aagacgaaca tctgcgaaag     840
   catttgccaa ggatgttttc attgatcaag aacgaaagtt gggggctcga agacgattag     900
   ataccgtcgt agtctcaacc ataaacgatg ccgactaggg attggcaggt gtttcgttga     960
   tgaccctgcc agcaccttat gagaaatcaa agtttttggg ttccgggggg agtatggtcg    1020
   caaggctgaa acttaaagga attgacggaa gggcaccacc aggcgtggag cctgcggctt    1080
   aatttgactc aacacgggga aacttaccag gtccagacac ggggaggatt gacagattga    1140
   gagctctttc ttgattctgt gggtggtggt gcatggccgt tcttagttgg tgggttgcct    1200
   tgtcaggttg attccggtaa cgaacgagac ctcagcctgc taaatagtca cgggtacctc    1260
   gtgtacacgc ttgacttctt agagggacta ttggcgttta gtcaatggaa gtgtgaggca    1320
   ataacaggtc tgtgatgccc ttagatgttc tgggccgcac gcgcgctaca ctgatgcatt    1380
   caacgagcct atccttgacc gagaggtccg ggtaatcttt gaaactgcat cgtgatgggg    1440
   atagattatt gcaattatta gtcttcaacg aggaatgcct agtaagcgca agtcatcagc 1500
   ttgcgttgat tacgtccctg ccctttgtac acaccgcccg tcgctcctac cgattggg 1558
<210> 11
   <211> 761
   <212> DNA
   <213> Coccomyxa actinabiotis
<220>
   <221> rRNA
   <222> (1)..(761)
   <223> Séquence ITS1-ARNr5.8S-ITS2
<220>
   <221> misc_feature
   <222> (305)..(305)
   <223> n is a, c, g, or t
<220>
   <221> misc_feature
   <222> (457)..(457)
   <223> n is a, c, g, or t
<400> 11
   ggcctgccgc tcggagtagt cgaggtgaac ctgcggaagg atcattgcat cgatcaaaca 60
   caaaccgcga accacgtatc cccgctgggc ggctcctctt ttgttatatc gaaggaaggc 120
   cgaacagtgc gcttgtccct gacccgggtc ctcggctttc gaggctcggc aggtctggcg 180
   ggccggggag acctctctcc gatcctgacc cgatcggatg acgtcttctg tcggtcggca 240
   aaacccccaa tcacaaccga acgtaacgaa gcctgaagca atcggcgtgc tgtcatcagc 300
   cgtcncgaaa ccaatgacaa ctctcaacaa tggatgtctt ggctcccgta tcgatgaaga 360
   acgcagcgaa atgcgatacg tagtgtgaat tgcagataat gtgaatcatc gaatctttga 420
   acgcaaattg cactcgaggc tccggccaag agtatgnctg cctcagcgtc ggctcacccc 480
   ctcgcctccc ctttgtctcc taccgaacaa ggaaggatgg cggacgtggc ggtcccggtg 540
   aactttcctt cgaaggacaa ccgggtccgc tgaagaccag aggctcgagc aaggacccat 600
   tcgggacaaa cagtctggta ggagcgtcgt ctcagacctc atgcccgctg tcggcccggg 660
   actttgttgg aggccggaag gcgctcctcg gttatggtaa ccggagctct tcatgaaata 720
   ttcgacctga gttcaggcaa gggcacccgc tgaacttaag c 761
<210> 12
   <211> 617
   <212> DNA
   <213> Coccomyxa chodatii souche SAG 216-2
<220>
   <221> rRNA
   <222> (1)..(617)
   <223> Séquence ITS1-ARNr5.8S-ITS2
<400> 12
   gtgaacctgc ggaaggatca ttgcatcgat caaacacaca ccgcgaaccc acgtatcccc      60
   gcgggcggct ccttttcctt cgtgagaagt cagccggctg tgcgcttgtc cctgccgcaa     120
   caccggcagg tctggcgggc cggggaggcc gctctcctct aaccgggaga ggacctaatc     180
   cggtcggcaa aaccccaatc gatcccaacc agaaaccgcc cgaagacgtc gacggctgtc     240
   tgacagccgt ctccaaccaa agacaactct caacaacgga tatcttggct cccgtatcga     300
   tgaagaacgc agcgaaatgc gatacgtagt gtgaattgca gataatgtga atcatcgaat     360
   ctttgaacgc aaattgcact cgaggctccg gccgagagta tgtctgcctc agcgtcggct     420
   ttaccctcac ccctctttca agaggcgtgg acctggccgt tccggttgac ttaaccgggt     480
   ctgccgaaga acagaggctc taacgaggac ccaatcggga cgactgtctg gtaggtgacc     540
   cgccagggat cgcatgccgc cgtcggcccg ggacctggtt gtaagccggc aggcaagaac     600
   acccgctgaa cttaagc                                                    617
<210> 13
   <211> 708
   <212> DNA
   <213> Coccomyxa peltigerae souche SAG 216-5
<220>
   <221> rRNA
   <222> (1)..(708)
   <223> Séquence ITS1-ARNr5.8S-ITS2
<400> 13
   gtgaacctgc ggaaggatca ttgcatcgat caaacacaaa ccgcgaaccc acgtatcccc      60
   gcggccttct gtctttctct tcggggaaag agagccggcc gtgcgcttgt ccctgtccgg     120
   ttccgcttcg gcggaaaggg caggtctagc gggctgggga ggccgctctc ccttaaccgg     180
   gggagggccg aaacccggct ggcaaaaacc ccattcgatc caaaaccaga aaataactga     240
   agatccggac agctgttcaa gaacagccgt ctccaaccaa agacaactct caacaacgga     300
   tatcttggct cccgtatcga tgaagaacgc agcgaaatgc gatacgtagt gtgaattgca     360
   gataatgtga atcatcgaat ctttgaacgc aaattgcact cgaggctccg gccgagagta     420
   tgtctgcctc agcgtcggct tcaccctcac ccctctccac cgagaggcgt ggacctggcc     480
   gtcccggtta atgcaaccgg gtctgccgaa gaacagaggc tcgaacgagg acccaatcgg     540
   gacaacagtc tggtaggtga cccgtcaggg atcgcatgcc gctgtcggcc cgggacctcg     600
   ttgtaagccg gcaggaatcg gcggtcgctt ttttttagaa aaaggagcgg ccgcacgaca     660
   cacccttttc gacctgagtt caggcaagaa cacccgctga acttaagc                    708

## Description

L'invention est relative à de nouvelles algues et à leur utilisation pour le captage de métaux à partir de milieux aqueux, et notamment de milieux radioactifs.

Des effluents radioactifs sont produits principalement par les centrales de production d'électricité nucléaire. Il s'agit principalement des eaux des piscines d'entreposage de combustible usé, des eaux des bassins de décontamination, ou encore des eaux des circuits de refroidissement des installations nucléaires, qui finissent par contenir des composés radioactifs, du fait de l'activation de composés inactifs par les rayonnements, ou du relargage et de la dissolution de composés radioactifs. D'autres sources d'effluents radioactifs sont la médecine nucléaire, les laboratoires de recherche utilisant des matériaux radioactifs, ainsi que certaines industries non nucléaires (par exemple l'extraction des terres rares).

Diverses méthodes physiques et chimiques sont utilisées pour purifier les effluents, notamment les eaux, contenant des composés radioactifs. Mais elles ont des coûts de fonctionnement et d'équipement importants, demandent une maintenance lourde et génèrent des volumes importants de déchets radioactifs. De plus, leur champ d'application est souvent limité. Par exemple, des résines échangeuses d'ions sont utilisées pour maintenir une faible conductivité dans les eaux des installations nucléaires. Elles se chargent en ions radioactifs et sont entreposées dans l'attente d'une filière de retraitement adaptée lorsqu'elles sont saturées ou stockées dans des conditions mettant en oeuvre des composés toxiques ou très réactifs.

Par ailleurs, il existe des méthodes biologiques utilisant par exemple des bactéries, des champignons, des levures ou des plantes pour purifier des milieux contaminés (effluents industriels, milieux naturels, etc.) par des produits toxiques (non radioactifs ou radioactifs). Ces méthodes utilisent des organismes vivants pour concentrer, assimiler, rendre moins toxiques (modification de la forme chimique) des composés polluants ou de la biomasse non-vivante ainsi que des dérivés provenant d'organismes vivants pour biosorber les polluants. Les méthodes biologiques ont en général un champ d'application plus large que les méthodes physiques et chimiques. Elles ne nécessitent pas l'ajout de réactif ou de produit chimique et permettent en général un traitement à plus faible coût, d'où leur intérêt économique.

Les plantes en particulier sont de bons épurateurs de sols ou d'eaux car elles possèdent tout un système de métabolites, protéines, enzymes, mécanismes d'import, canaux membranaires, structure interne, etc... qui les rendent capables selon les cas d'immobiliser des composés toxiques, de les chélater à l'extérieur ou à l'intérieur de la plante, de les incorporer en plus ou moins grande quantité via des voies d'import spécifiques ou non, de les séquestrer à l'intérieur des cellules, de modifier leur spéciation pour les rendre inoffensifs ou moins toxiques, de les rendre moins solubles, de les stocker sous forme non toxique dans la vacuole, etc.

Des études ont montré que certains micro-organismes étaient capables de concentrer par biosorption des ions métalliques, tels que Ag, Al, Au, Co, Cd, Cu, Cr, Fe, Hg, Mn, Ni, Pb, Pd, Pt, U, Th, Zn, etc, dans des solutions diluées (White et al., International Biodeterioration & Biodegradation, 35 : 17-40, 1995 ; Brevet US 6355172). La biosorption est la capacité de la biomasse à fixer des métaux lourds au moyen de mécanismes physico-chimiques non sélectifs par des interactions avec les groupements fonctionnels de composés pariétaux situés à la surface des cellules. Par exemple, il a été proposé d'utiliser des bactéries et des mélanges de micro-organismes pour biosorber non sélectivement des métaux lourds (Brevet US 7,479,220 ; Demande PCT WO 03/011487).

D'autres méthodes utilisent de la biomasse morte ou des composés dérivés provenant de la culture d'organismes vivants pour dépolluer des milieux contaminés en métaux. Les processus mis en jeu sont des mécanismes physico-chimiques, inactifs biologiquement, tels l'échange d'ions par exemple avec les polysaccharides présents dans les parois cellulaires, la complexation, ou l'adsorption.

La biomasse dérivée d'algues (par exemple des parois cellulaires) a été utilisée pour épurer des métaux contenus dans des effluents liquides (Brevet US 4769223 ; Demande PCT WO 86/07346 ; Brevet US 5648313 ; Demande PCT WO 2006/081932).

Pour le traitement de milieux pollués par des composés radioactifs, les méthodes faisant appel à des organismes vivants sont peu nombreuses. En effet, dans le cas d'eaux contaminées par des composés radioactifs ou d'eaux situées au voisinage de sources radioactives, il est nécessaire d'utiliser des organismes radiotolérants ou radiorésistants, qui soient de plus capables de résister à la toxicité chimique des contaminants et de fixer les composés d'intérêt en quantité suffisante pour être utilisés dans le cadre d'un procédé industriel.

En milieu naturel, les organismes accumulant des composés radioactifs sont en général soumis à une radioactivité faible. Par exemple, immédiatement après l'accident de Tchernobyl, pour les milieux aquatiques, le débit de dose de rayonnements ionisants externe de l'eau du réservoir de refroidissement du réacteur n'a pas excédé 100 µGy/h et la dose maximale cumulée sur 1 an était de 0,01 Gy en 1986. Le débit de dose provenant des radionucléides déposés sur les sédiments dans la rivière Pripyat, située dans la zone de 30 km autour de la centrale, est quant à lui monté ponctuellement à 0,4 mGy/h immédiatement après l'accident (Kryshev et Sazykina, Journal of Environmental Radioactivity, 28 : 91-103, 1995).

Dans la plupart des cas, la résistance aux rayonnements ionisants des microorganismes proposés pour dépolluer des matériaux ou des effluents contaminés par la radioactivité et/ou concentrer des composés radioactifs n'a pas été testée. En effet, ils sont utilisés pour extraire U (uranium) et Th (thorium) dont l'activité des principaux isotopes est faible (par exemple pour ²³⁸U ou ²³⁵U, l'activité est de 0,13 ou 0,8 Bq/l pour une solution contenant 10 µg/l de ²³⁸U ou ²³⁵U, respectivement.

Le Brevet US 4320093 propose l'utilisation de champignons du genre *Rhizopus* pour extraire de l'uranium ou du thorium contenu dans des effluents aqueux. Le Brevet GB 1472626 propose l'utilisation d'algues vertes unicellulaires mutantes obtenues par irradiation aux rayons X d'algues vertes unicellulaires préalablement accoutumées à l'uranium, et le Brevet GB 1507003 propose l'utilisation de divers microorganismes, notamment le champignon *Aspergillus niger*, et des Cyanobactéries de type *Oscillatoria*, pour concentrer l'uranium naturellement présent dans l'eau de mer. Le Brevet US 7,172,691 propose l'utilisation d'algues vivantes photosynthétiques des genres *Chlorelle*, *Scenedesmus*, *Oocystis*, et *Chlamydomonas*, pour concentrer des contaminants radio-actifs, et notamment de l'uranium, à partir de milieux aqueux contenant une concentration d'uranium de l'ordre de 0-20 ppm, ce qui représente une activité de 260 et 1600 Bq/l pour ²³⁸U et ²³⁵U, respectivement. En comparaison, l'activité de l'eau des piscines d'entreposage d'éléments nucléaires qui constituent le milieu de vie de la micro-algue décrite ci-après est d'environ 300 000 Bq/l.

Les organismes les plus radiorésistants décrits jusqu'à présent sont des procaryotes. L'espèce *Deinococcus radiodurans* possède une extraordinaire capacité de résistance aux rayonnements ionisants, se développe sous des irradiations de 60 Gy/h et survit à des doses de 15 kGy. Cependant, cette bactérie est naturellement peu résistante aux métaux. Par exemple, elle ne tolère pas le cobalt (quel que soit l'isotope) qui inhibe sa croissance à 5 ppm soit environ 10⁻⁴ mol/l (John et al., Symposium Chemical-biological Interactions In Contaminant Fate, Metal Toxicity In Deinococcus radiodurans, p. 426-428 in Preprints of Extended Abstracts Vol. 40 No. 2, 2000). Son utilisation comme épurateur des métaux contenus dans des milieux radioactifs nécessite donc des modifications génétiques pour introduire des gènes qui permettront d'accumuler les métaux d'intérêt. Ainsi, a t-il été proposé de modifier génétiquement des bactéries du genre *Deinococcus* pour exprimer des enzymes capables de détoxifier ou de métaboliser des composés organiques, des métaux, ou des radionucléides, à des fins de bioremédiation *in situ* de sites de déchets nucléaires (Demande PCT WO 01/23526). Plus récemment, des bactéries de l'espèce *Kineococcus radiotolerans* ont été isolées et purifiées à partir d'un site de déchets radioactifs de haute activité. Ces bactéries ont été décrites comme étant capables de dégrader des contaminants organiques en présence de rayonnements ionisants dont le débit de dose est supérieur à 10 Gy/h, et leur utilisation pour la dépollution non sélective de radionucléides par biosorption a été proposée (Brevet US 7,160,715).

Bien que pouvant être utilisés pour décontaminer des milieux dont la radioactivité est très importante, ces deux microorganismes présentent l'inconvénient d'être non-autotrophes, et donc de nécessiter l'apport extérieur de nutriments carbonés pour pouvoir être utilisés sous forme de cultures vivantes. D'autre part, leur culture est plus sensible à la contamination par d'autres bactéries que celle d'organismes autotrophes, qui nécessitent par ailleurs un milieu de culture moins riche.

Une seule forme d'organisme autotrophe radiotolérant a été décrite (Farhi *et al*., 20 : 104216, 2008). Il s'agit d'une microalgue de la classe des Chlorophycées, qui tolère des rayonnements ionisants avec une DL50 de 6 kGy, alors qu'en général, les DL50 de résitance aux rayonnements ionisants des algues sont comprises entre 30 et 1200 Gy (AIEA, 1976, Effects of ionizing radiation on aquatic organisms and ecosystems. Technical reports séries No. 172, International Atomic Energy Agency, Vienna).

Les Inventeurs ont maintenant isolé une autre microalgue radiotolérante, appartenant au genre *Coccomyxa* (classe des Trebouxiophycées : Pröschold et Leliaert, Unravelling the algae: the past, present, and future of algal systematics, CRC Press, Brodie and Lewis eds., 2007), et ont découvert que non seulement cette algue présentait une résistance aux rayonnements ionisants encore plus importante que celle de la microalgue décrite par Farhi et al. (2008, ci-dessus), mais qu'en outre elle était capable de capter et de concentrer des ions métalliques, radioactifs ou non, en solution dans un milieu aqueux, et qu'elles pouvaient se développer en milieu radioactif.

Cette algue représente une nouvelle espèce de *Coccomyxa*, dénommée ci-après *Coccomyxa actinabiotis.*

Une culture axénique représentative de cette nouvelle espèce a été déposée selon le traité de Budapest le 23/06/2009 auprès de la Culture Collection of Algae and Protozoa (CCAP), Scottish Association for Marine Science, Dunstaffnage Marine Laboratory, GB-Oban, Argyll, PA37 1QA, UK, sous le numéro CCAP 216/25.

Les algues de l'espèce *Coccomyxa actinabiotis* se caractérisent notamment en ce que leurs gènes correspondant à l'ARN ribosomique 18S-ITS1-ARN ribosomique 5.8S-ITS2-ARN ribosomique 26S (début) contiennent une séquence présentant au moins 95%, et, par ordre de préférence croissante, au moins 96%, 97%, 98%, ou 99% d'identité avec la séquence SEQ ID NO: 1.

Le pourcentage d'identité indiqué ci-dessus est calculé après alignement des séquences en utilisant l'algorithme Clustal (Larkin et al., Bioinformatics, 23, 2947-2948, 2007), sur une fenêtre de comparaison constituée par la totalité de la séquence SEQ ID NO: 1.

Les algues de l'espèce *Coccomyxa actinabiotis* peuvent également être caractérisées en ce que la région correspondant aux ITS1-ARNr5.8S-ITS2 présente au moins 90% d'identité avec la région correspondante de la séquence SEQ ID NO: 1. Ce seuil a été estimé à 90% sur la base des observations des Inventeurs, qui montrent un maximum de 81% d'identité entre cette région chez *Coccomyxa actinabiotis* et chez d'autres *Coccomyxa* ainsi qu'un maximum de 88% d'identité entre les ITS1-ARNr5.8S-ITS2 des autres *Coccomyxa* comparés entre eux (cf. Tableau III ci-après).

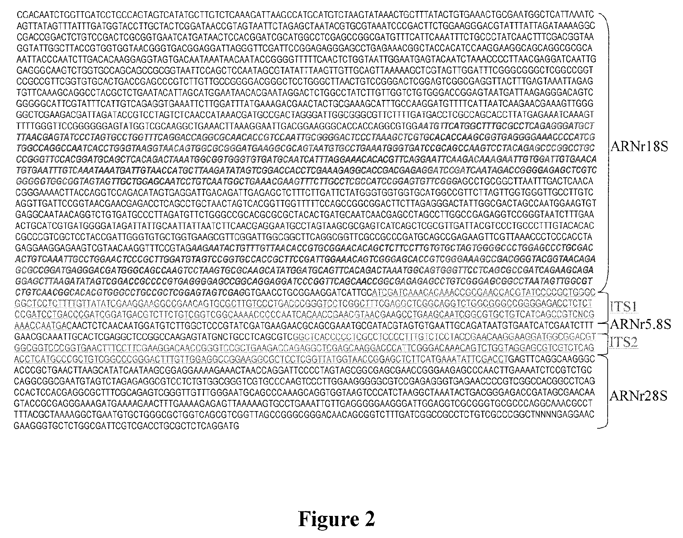

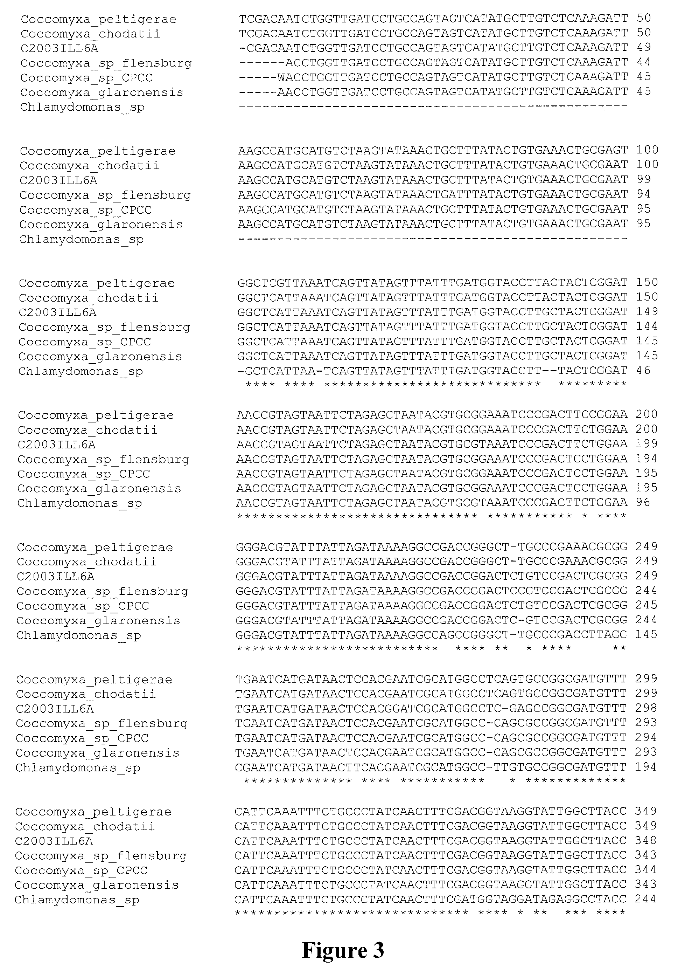

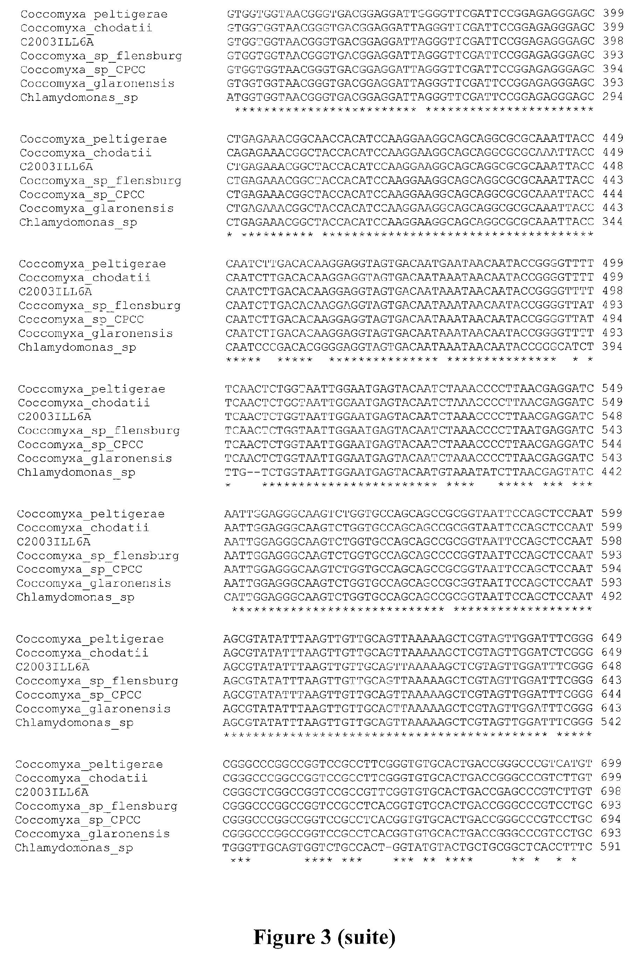

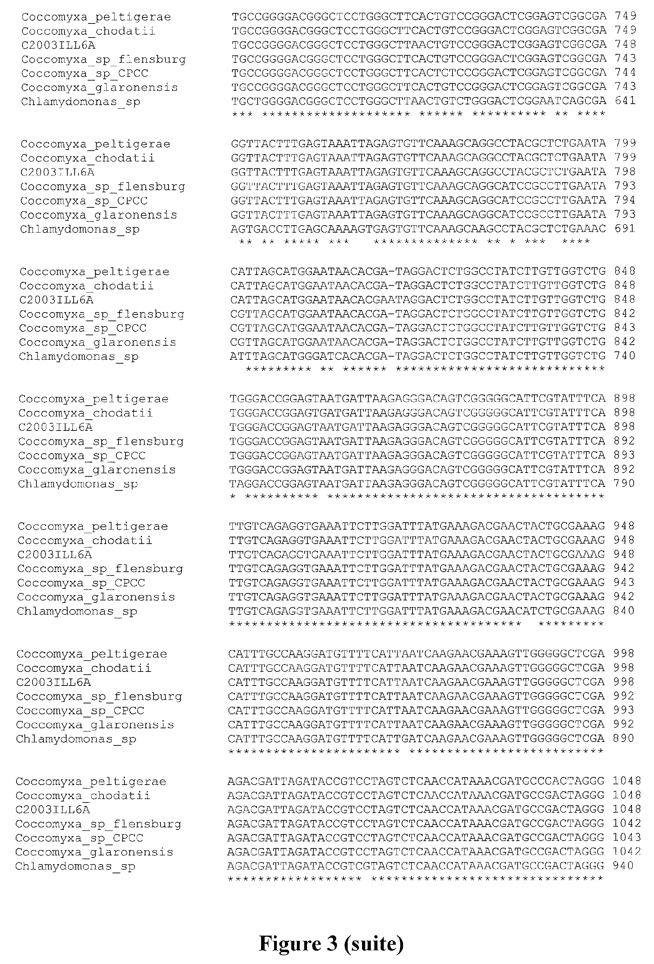

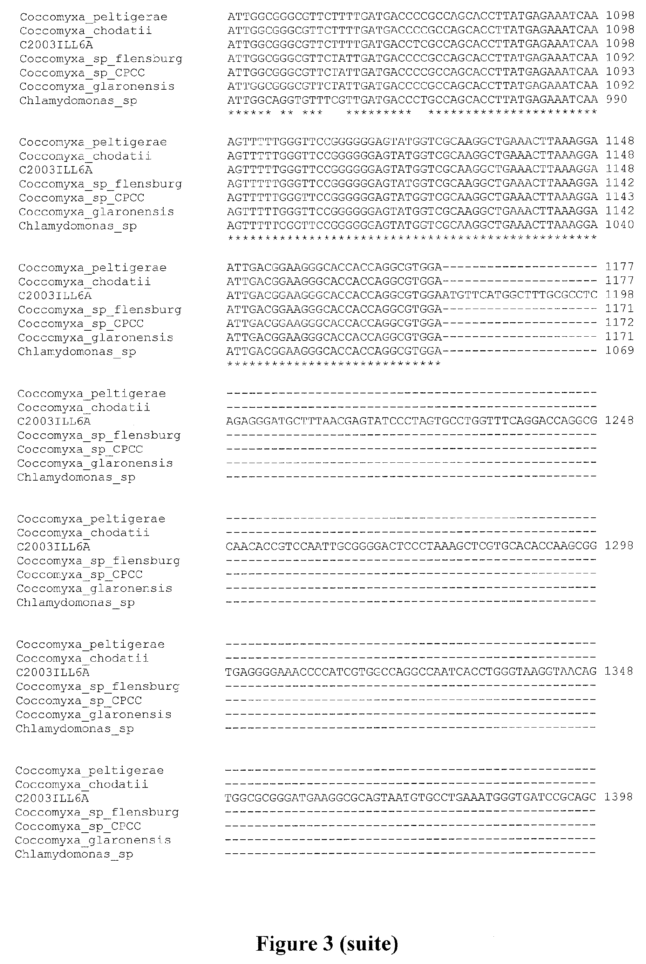

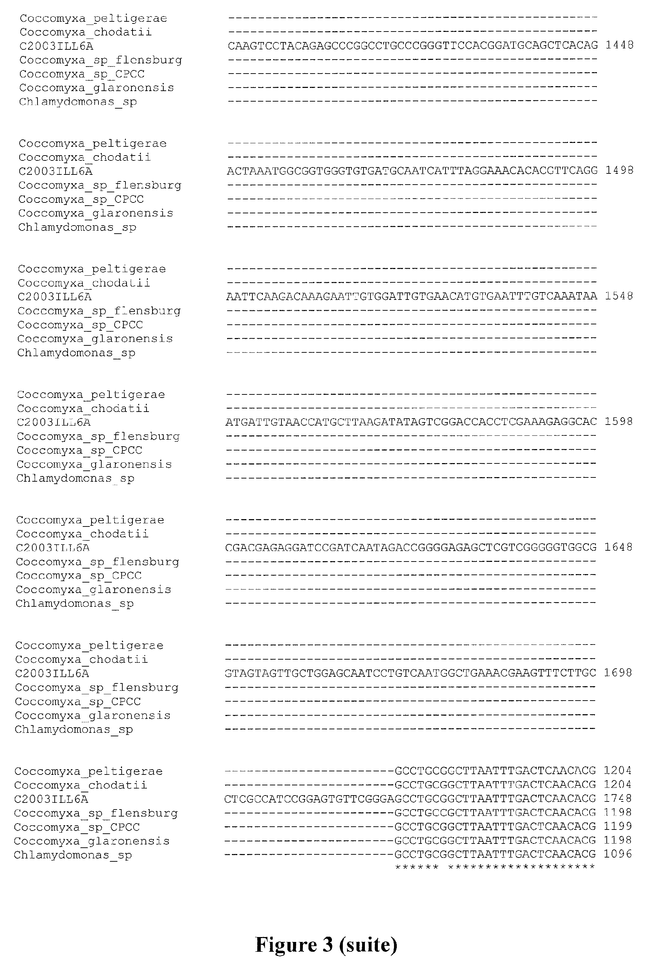

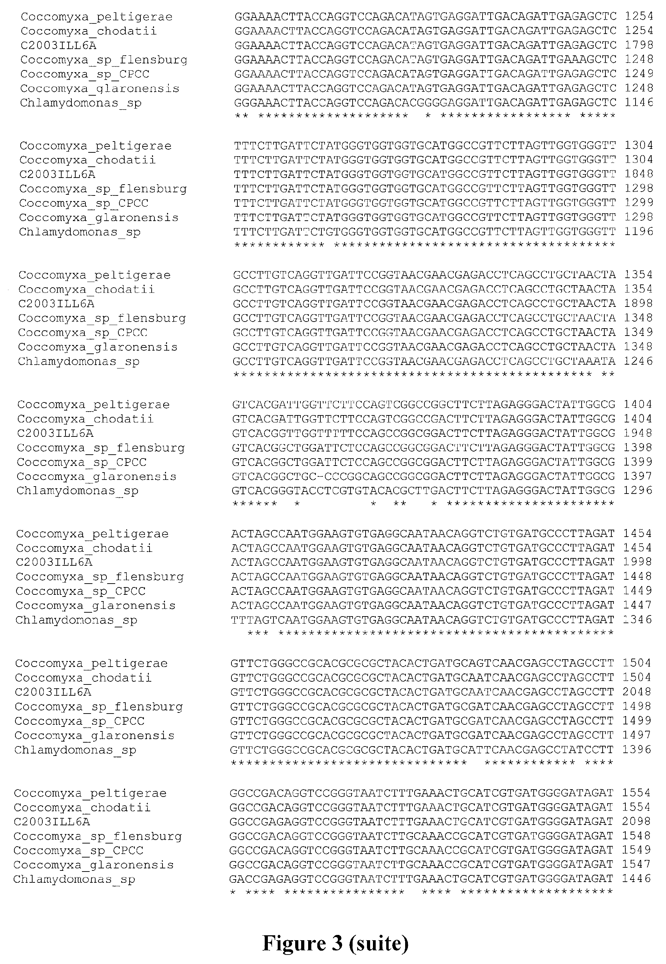

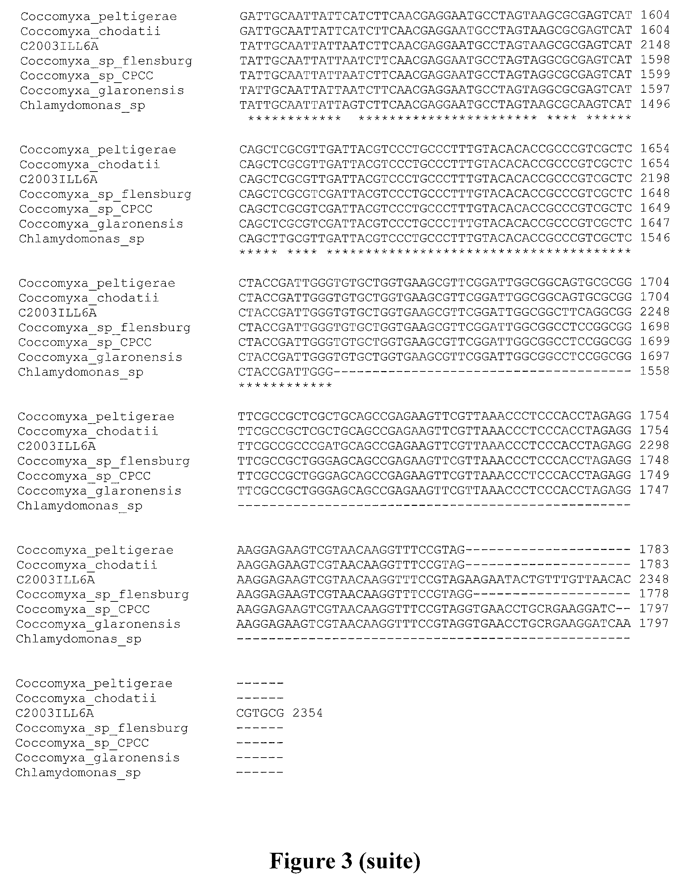

La séquence des gènes d'ARN ribosomique de *Coccomyxa actinabiotis* se différencie notamment de celle d'autres espèces du genre *Coccomyxa* par la présence, dans la région correspondant à l'ARNr18S de 2 inserts de 500 pb environ (inserts représentés en italique sur la Figure 2). Elle se différencie aussi par la nature de son gène d'ARNr 18S (Figure 3), et par celle de ses ITS1 et ITS2.

*Coccomyxa actinabiotis*, de par sa résistance aux rayonnements ionisants, peut croître en milieu radioactif, ce qui lui permet de capter et métaboliser des composés radioactifs autres que des métaux (par exemple ³H ou ¹⁴C), en plus de pouvoir capter et concentrer des ions métalliques, radioactifs ou non, en solution dans un milieu aqueux.

La présente invention a en conséquence pour objet l'utilisation d'algues vertes de l'espèce *Coccomyxa actinabiotis* définie ci-dessus, pour capter au moins un élément choisi parmi les métaux Ag, Co, Cr, Zn, Mn, Sb, Cs, Ni, Fe, actinides, ou lanthanides, qu'ils soient radioactifs ou non, ou les radioisotope ¹⁴C et ³H à partir d'un milieu aqueux contenant ledit métal ou ledit radioisotope en solution.

Plus particulièrement la présente invention a pour objet un procédé de captage d'au moins un élément choisi parmi les métaux, radioactifs ou non, Ag, Co, Cr, Zn, Mn, Sb, Cs, Ni, Fe, actinides, ou lanthanides, ou les radioisotopes ¹⁴C et ³H à partir d'un milieu aqueux contenant ledit élément en solution caractérisé en ce que ledit captage est effectué en incubant des algues vertes du genre *Coccomyxa* dans ledit milieu aqueux.

De manière avantageuse, ledit élément est un métal choisi parmi Ag, Co, et l'actinide U.

De manière également avantageuse, ledit élément est ¹⁴C. La quantité de ¹⁴C dans les rejets des installations nucléaires en France n'est réglementée de manière individuelle que depuis quelques années. Or le ¹⁴C est le principal polluant radioactif émis dans l'environnement par les installations nucléaires, après le tritium. Par exemple, les installations nucléaires de base du centre nucléaire de production d'électricité du Tricastin (4 tranches de 900 MW) ont rejeté 170 GBq de ¹⁴C dans l'atmosphère et 15,9 GBq de ¹⁴C dans les effluents liquides en 2009 (Rapport Annuel EDF 2009). Les usines de retraitement utilisant le procédé PUREX® (par exemple, AREVA La Hague) rejettent dans l'environnement environ 600 GBq/an de ¹⁴C pour le retraitement de la totalité du combustible d'un réacteur de 1 GWe (Toxicologie nucléaire environnementale et humaine, eds Tec et Doc, Lavoisier, 2009).

Selon un mode de mise en oeuvre préféré de la présente invention, ledit milieu aqueux est un milieu radioactif, c'est-à-dire un milieu dans lequel lesdites algues sont soumises à un débit de dose pouvant aller de quelques µGy/h jusqu'à 1 kGy/h. Selon une disposition préférée de ce mode de mise en oeuvre, l'élément à capter est un métal choisi parmi ceux indiqués ci-dessus, sous forme d'isotope radioactif, ou sous forme de mélange d'isotopes.

La durée d'incubation des algues dans le milieu aqueux peut varier notamment selon le ou les éléments concerné(s), d'une part, et la nature du milieu aqueux à partir duquel le captage doit être effectué, d'autre part. Elle sera généralement d'au moins 1 heure, et peut aller jusqu'à plusieurs mois, voire plusieurs années. Par exemple, si l'on souhaite capter uniquement Ag, une durée d'incubation d'environ 1 heure pourra être suffisante pour en capter la majeure partie et d'environ 10 à 20 heures pour en capter la quasi totalité ; si d'autres métaux doivent être captés, une durée d'incubation plus longue, d'au moins 3 à 5 jours, et avantageusement d'au moins une semaine pourra être utilisée. Autre exemple, si l'on souhaite capter le ¹⁴C, une durée d'incubation de 3 h, dans les conditions optimisées proposées dans l'exemple 4 ci-après, suffira pour en capter la majeure partie.

La durée maximale d'incubation qui peut être envisagée dépendra en fait principalement de la capacité de croissance et de survie des algues dans le milieu aqueux.

En présence de lumière et de gaz carbonique (introduit par contact avec l'air ambiant, agitation des cultures ou bullage), les algues du genre *Coccomyxa*, et notamment de l'espèce *Coccomyxa actinabiotis*, peuvent croître et vivre pendant de très longues périodes dans de l'eau faiblement minéralisée (conductivité 1 à 1,5 µS/cm), à un pH de 5 à 6 et une température de 20 à 30°C, et dans l'eau déminéralisée (conductivité de 0,05 µS/cm), elles peuvent croître et vivre pendant 3 à 4 semaines. Ces algues vertes ayant besoin de lumière pour réaliser la photosynthèse et fabriquer leur matière organique, leur croissance cesse lorsqu'elles sont placées à l'obscurité.

Ainsi, selon un mode de mise en oeuvre du procédé conforme à la présente invention, la croissance des algues vertes de l'espèce *Coccomyxa actinabiotis* peut être maîtrisée en contrôlant l'illumination du milieu aqueux comprenant lesdites algues.

Les algues *Coccomyxa actinabiotis* peuvent en outre croître et vivre pendant plusieurs années dans un milieu faiblement radioactif, où elles sont soumises à une irradiation inférieure ou égale à 0,15 mGy/h. Elles peuvent résister jusqu'à environ 1 mois à une irradiation de 1 Gy/h. Elles peuvent également résister pendant environ une journée à une irradiation de 300 Gy/h et environ 3 jours à une irradiation de 100 Gy/h. En milieu très fortement radioactif, elles peuvent résister jusqu'à 20 heures à une irradiation 1 kGy/h ; si elles sont ensuite transférées dans un milieu non-radioactif, elles récupèrent en moins de deux semaines leur capacité de croissance. A titre indicatif, la limite du domaine des faibles débits de dose, en dessous de laquelle aucun effet biologique n'a été détecté, peut être placée vers 1 mSv/h (soit 1 mGy/h pour un rayonnement γ). Une faible dose correspond à une dose inférieure à 10 mSv. Un débit de dose supérieur à 1 mSv/h produit des effets biologiques dangereux. Pour des doses supérieures à 10-100 mSv, des effets statistiquement observables apparaissent. Une forte dose correspond à une irradiation supérieure à 1 Gy, valeur à partir de laquelle on commence à voir apparaître un effet déterministe.

Pour la mise en oeuvre du procédé conforme à l'invention, les algues peuvent être utilisées en suspension dans le milieu aqueux à partir duquel le captage doit être effectué, sous agitation pour éviter leur agglomération. Elles peuvent également être fixées sur un support solide, lisse, poreux, ou sous forme de billes, placé dans ledit milieu aqueux.

Le procédé peut être déporté, c'est-à-dire que les algues sont mises en contact avec le milieu à dépolluer dans une enceinte séparée de l'installation nucléaire, ou mis en oeuvre *in situ*; dans ce dernier cas, les algues sont alors implantées directement dans le milieu à dépolluer.

Dans le cas d'un procédé de captage ou de décontamination *in situ*, les algues peuvent séjourner dans l'installation tant qu'elles ne gênent pas les opérations. A titre indicatif, leur croissance peut être maîtrisée par l'intensité d'éclairement (obscurité ou faible illumination), ou le choix de la longueur d'onde des lampes (par exemple lumière inactinique jaune-vert). Une filtration des eaux permet également de maîtriser leur croissance en captant les algues en suspension dans l'eau. S'il s'avère nécessaire de les éliminer, la destruction définitive des algues pourra s'accompagner d'un relargage des métaux, et devra donc préférentiellement s'effectuer par section dans les installations, avec récupération des effluentes concentrés en métaux.

Dans le cas d'un procédé déporté, à la fin de l'incubation, les algues ayant capté les métaux sont récoltées par des moyens conventionnels (filtration, décantation, centrifugation, etc.). Elles peuvent ensuite être éliminées comme des déchets éventuellement après avoir été séchées et/ou brûlées, sans extraction préalable des métaux qu'elles contiennent.

Avantageusement, on peut récupérer les métaux captés par les algues afin de recycler ces métaux.

Cette récupération peut s'effectuer par tous moyens appropriés. Il est par exemple possible de récupérer, de manière non-destructive, au moins une partie des métaux captés par les algues, ce qui permet le cas échéant de réutiliser celles-ci, en particulier pour les métaux tels Ag et Cr qui après captage restent en grande partie au niveau du mucilage et de la surface cellulaire. Cette récupération peut se faire en mettant les algues en présence d'une solution complexante ou acide. Ainsi, elle peut notamment se faire pour des métaux tels que Ag, par traitement des algues en milieu acide, à un pH de 1 à 2,5, de préférence de 1,5 à 2 pendant 1 à 2 jours.

Pour d'autres métaux, tels que Cr, mais aussi pour la portion de métaux tels que Co qui est fixée au niveau du mucilage et de la surface cellulaire, elle peut également se faire par un traitement par un agent chélatant, tel que l'EDTA à 0,1 mol/l pendant 1 h à 3 jours, de préférence pendant environ 1 jour.

La récupération peut se faire pour les métaux tels que Co qui, après captage par les algues, sont séquestrés à l'intérieur des cellules, par traitement des algues en milieu acide, à un pH de 0,5, pendant 2 à 10 jours, mais sans garantie de la viabilité ultérieure des cellules.

Les métaux peuvent également être récupérés après destruction des algues. Cette destruction peut par exemple être effectuée par lyse des algues. Elle peut aussi être effectuée par incinération des algues.

Le procédé conforme à l'invention peut être mis en oeuvre dans tous les cas où l'on souhaite extraire des métaux, radioactifs ou non, et notamment ceux mentionnés ci-dessus, d'un milieu aqueux, à des fins d'exploitation minière, ou de dépollution d'effluents aqueux contenant des métaux, notamment d'effluents radioactifs.

Outre leur forte capacité à capter et concentrer spécifiquement les métaux mentionnés ci-dessus (Ag, Co, Cr, Zn, Mn, Sb, Cs, Ni, Fe, les actinides, ou les lanthanides, qu'ils soient radioactifs ou non), les algues du genre *Coccomyxa*, et notamment de l'espèce *Coccomyxa actinabiotis*, possèdent également des propriétés de captage non-spécifique d'autres métaux, notamment du fait de leur mucilage extra-cellulaire constitué de polysaccharides, qui ont la propriété de complexer les cations.

Le procédé conforme à l'invention est donc particulièrement approprié à la dépollution et à la décontamination de milieux aqueux ou de sols (tourbières, marais) contaminés par des métaux, radioactifs ou non, et plus particulièrement de milieux radioactifs, tels que l'eau des piscines de stockage ou encore l'eau légère des circuits de refroidissement secondaires de centrales ou de réacteurs nucléaires, ou les effluents de centrales nucléaires rejetés dans l'environnement.

La présente invention peut être mise en oeuvre non seulement avec des algues vertes unicellulaires de l'espèce *Coccomyxa actinabiotis*, mais également avec un mélange de microorganismes comprenant des algues vertes unicellulaires de l'espèce *Coccomyxa actinabiotis* et au moins un microorganisme, notamment une bactérie, un champignon, une levure, une autre algue unicellulaire, et/ou une plante multicellulaire, de préférence radiorésistant ou radiotolérant, capable de concentrer des ions métalliques en solution et/ou de capter et métaboliser des composés radioactifs autres que des métaux (par exemple ³H ou ¹⁴C). Les microorganismes et plantes multicellulaires utilisables en combinaison avec les algues vertes unicellulaires de l'espèce *Coccomyxa actinabiotis* sont notamment ceux et celles cités ci-dessus, en particulier ceux et celles étant radiorésistants ou radiotolérants. Dans le cas où la présente invention est appliquée à un milieu aqueux radioactif, le temps d'incubation du mélange de microorganismes dépendra de la résistance individuelle des microorganismes composant le mélange. De même, les conditions de culture pourront être adaptées afin de favoriser la croissance d'un ou plusieurs microorganismes constituant le mélange.

La présente invention sera mieux comprise à l'aide du complément de description qui va suivre, qui se réfère à des exemples illustrant l'isolement et la caractérisation de l'espèce *Coccomyxa actinabiotis*, ainsi que son utilisation pour décontaminer un milieu aqueux radioactif. Les exemples sont illustrés à l'aide des figures suivantes :
- Les Figures 1A et 1B représentent des photographies de micro-algues selon l'invention observées en microscopie photonique (microscope Zeiss binoculaire Axioplan 2, grossissement 1000 et 3000 pour les Figures 1A et 1B, respectivement). La Figure 1B montre l'accolement de deux cellules flagellées.
- La Figure 2 montre la séquence des gènes de l'ADN ribosomal ARNr18S-ITS1-ARNr5.8S-ITS2-ARNr26S (500 premières bases) des micro-algues *Coccomyxa actinabiotis.*
- La Figure 3 montre la comparaison des séquences correspondant à l'ARN de la petite sous-unité du ribosome (ARNr 18S) de la micro-algue CCAP 216/25, et des autres espèces de *Coccomyxa* répertoriées sur les bases de données (après avoir retiré les 2 inserts spécifiques de la séquence SEQ ID NO: 1), réalisée par alignement multiple des séquences au moyen du logiciel ClustalW2 2.0.12.
- La Figure 4 montre le pourcentage de mortalité de *Coccomyxa actinabiotis* en fonction de la dose d'irradiation en gray (Gy).
- La Figure 5 illustre la fixation et concentration de métaux par *Coccomyxa actinabiotis.* Les facteurs de concentration, définis comme le rapport de la concentration du métal adsorbé ou absorbé dans les micro-algues (en atomes/g de matière fraîche) à la concentration du métal dans l'eau (atomes/ml), obtenus pour les métaux ^{110m}Ag, ⁶⁰Co, ⁵¹Cr, ⁶⁵Zn, ⁵⁴Mn et ¹²⁴Sb, sont représentés.

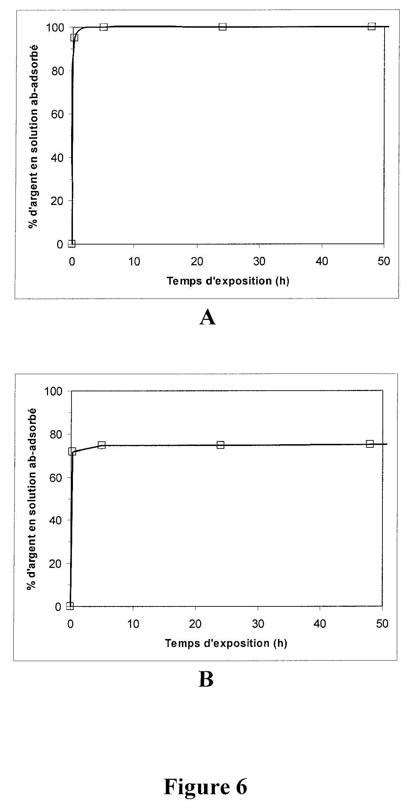

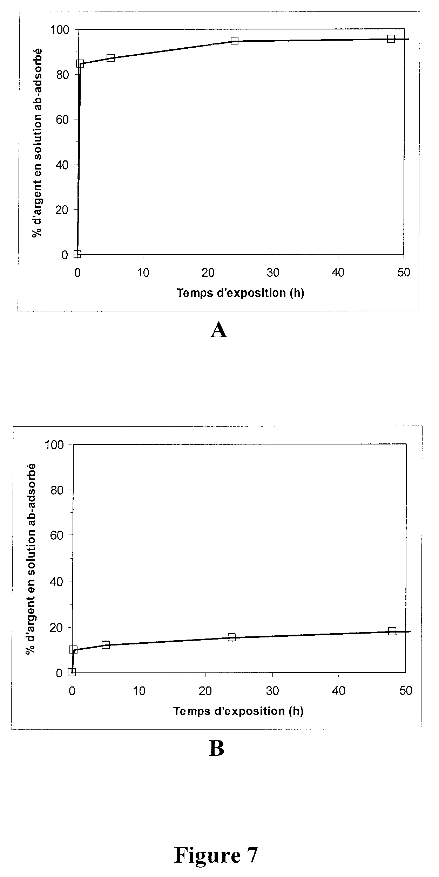

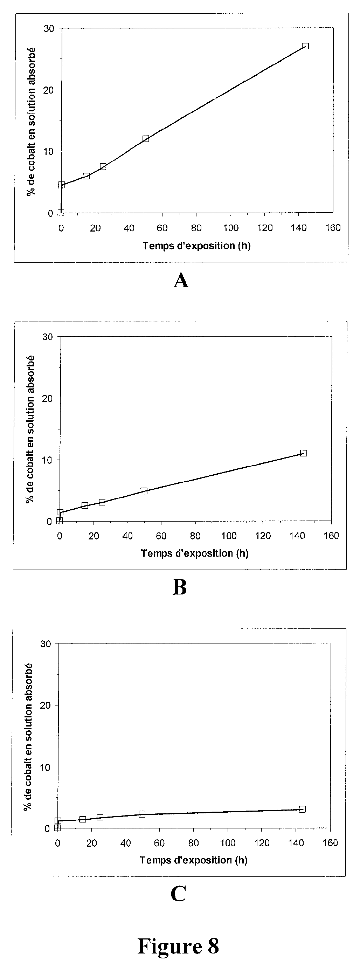

- Les Figures 6 à 8 montrent les cinétiques de fixation et d'incorporation de métaux, argent (Figures 6 et 7) et cobalt (Figure 8), par les micro-algues *Coccomyxa actinabiotis.* avec (Figure 7) ou sans (Figures 6 et 8) mucilage. Les quantités de métal ad-absorbé, en pourcentage, sont représentées en fonction du temps d'exposition (en heure). Figure 6 A: solution contenant 110 µg/l d'ions argent; Figure 6B : solution contenant 5,5 mg/l d'ions argent. Figure 7A : solution contenant 1,1 mg/l d'ions argent ; Figure 7B : solution contenant 55 mg/l d'ions argent ; Figure 8A : 19 µg/l d'ions cobalt ; Figure 8B : 0,94 mg/l d'ions cobalt ; Figure 8C : 4,8 mg/l d'ions cobalt.
- La Figure 9 montre l'incorporation du ¹⁴C par une culture de *Coccomyxae actinabiotis*. Le pourcentage de ¹⁴C sous forme d'hydrogénocarbonate, dans le culot (triangles), qui contient les algues, et dans le surnageant de culture (losanges), est représenté en fonction du temps, en heures.
- La Figure 10 représente la concentration en argent 110m, exprimée en kBq/l, dans une piscine d'entreposage d'éléments radioactifs, en fonction du temps (en jours), en présence de *Coccomyxa actinabiotis*.

### EXEMPLE 1: ISOLEMENT ET CARACTÉRISATION DE COCCOMYXA ACTINABIOTIS

La micro-algue a été récoltée dans une piscine de stockage de combustibles usés d'un réacteur nucléaire. L'eau contenue dans cette piscine a un pH 5,2 à 5,8, une conductivité de 1 à 1,5 µS/cm, est en contact avec l'air ambiant et contient des éléments radioactifs dissous. Sa température varie entre 23 et 30°C et est en moyenne de 25°C. L'activité radiologique dans la piscine est très variable selon les points de mesure, faible à très forte. L'activité gamma peut atteindre plusieurs milliers de Gy/h au contact d'éléments combustibles usés.

La présence de films de matière organique verte a été observée sur les parois et diverses surfaces de cette piscine. Des prélèvements ont été effectués et leur observation au microscope a montré qu'il s'agissait d'une micro-algue verte unicellulaire.

### Conditions de culture

Les échantillons prélevés ont été stockés et mis en culture à la lumière, à un pH de 5 à 6,5, et une température de 23°C, dans des erlemeyers stériles fermés par des bouchons poreux permettant les échanges gazeux.

Les milieux suivants ont été testés :
- le milieu de culture BBM (Bold's Basal Medium, SIGMA) pur ou dilué dans de l'eau déminéralisée. Le milieu BBM est classiquement utilisé pour la culture d'algues vertes.
- le milieu de culture BG11 (Rippka et al., « The Prokaryotes », Vol. 1 : 212-220, 1979 ; SIGMA). Ce milieu est classiquement utilisé pour la culture de cyanobactéries.
- de l'eau faiblement minéralisée (conductivité de 1 à 1,5 µS/cm), ou déminéralisée (conductivité de 0,05 µS/cm).

La composition des milieux de culture BBM et BG11 est indiquée dans le Tableau I ci-après :

**Tableau I**

| Constituants en g/L | BBM | BG 11 |
|---|---|---|
| NaNO3 | 0,25 | 1,5 |
| KH2PO4 | 0,175 | |
| K2HPO4 | 0,075 | |
| K2HPO4, 3H2O | | 0,04 |
| MgSO4, 7H2O | 0,075 | 0,075 |
| FeSO4, 7H2O | 0,005 | |
| CaCl2, 2H2O | 0,025 | 0,036 |
| NaCl | 0,025 | |
| Na2EDTA | 0,01 | |
| KOH | 0,006 | |
| ac citrique | | 0,006 |
| citrate d'ammonium ferrique | | 0,006 |
| Oligoéléments en mg/L | | |
| H3BO4 | 12,86 | 2,86 |
| MnCl2, 4H2O | 1,81 | 1,81 |
| ZnSO4, 7H2O | 0,222 | 0,222 |
| Na2MoO4, 2H2O | 0,39 | 0,39 |
| CuSO4, 5H2O | 0,079 | 0,079 |
| Co(NO3)2, 6H2O | 0,049 | 0,049 |
| pH | 6,4 | 6,5 |

Dans les milieux BG11 et BBM, les micro-algues croissent avec une phase de croissance exponentielle similaire dans les deux milieux.

Dans l'eau faiblement minéralisée, ou dans l'eau déminéralisée de conductivité de 0,05 µS/cm, les micro-algues poussent avec une phase de croissance rapide à court terme, voisine de celle obtenue dans les milieux BG11 et BBM, mais dans l'eau déminéralisée de conductivité de 0,05 µS/cm, leur santé se détériore au bout de 3 à 4 semaines, probablement du fait de l'épuisement des réserves.

Des algues ont été mises en culture sur un milieu de culture BBM solide gélosé. Des colonies circulaires ont ainsi été isolées, puis étalées individuellement sur milieu de culture gélosé. Cette opération a été répétée six fois afin d'obtenir une culture pure provenant d'une seule cellule.

Un échantillon de cette culture (dénommée ci-après micro-algue CCAP 216/25) a été déposé selon le traité de Budapest le 25 juin 2009 auprès de la Culture Collection of Algae and Protozoa (CCAP), Scottish Association for Marine Science, Dunstaffnage Marine Laboratory, GB-Oban, Argyll, PA37 1QA, UK, sous le numéro CCAP 216/25.

### Caractéristiques morphologiques et biochimique

Les micro-algues isolées sont unicellulaires. Les cellules, observées en microscopie photonique et en microscopie confocale, sont nucléées et de forme ovale. Leur longueur moyenne est de 6,8 ± 0,9 µm et leur largeur moyenne de 3,8 ± 0,6 µm. Toutefois, selon leur stade de croissance, il est possible d'observer des longueurs moyennes plus grandes, jusqu'à 9-10 µm, ou plus petites, de l'ordre de 5 µm, notamment juste après division.

Elles renferment un chloroplaste (peut-être plusieurs) qui contient la chlorophylle et qui est le siège de la photosynthèse. Un granule protéique, le pyrénoïde, occupe une partie du chloroplaste. Il intervient, entre autres, dans la synthèse de l'amidon. D'autres organites, notamment la vacuole, occupent le reste de la cellule.

Sous sa forme végétative, la cellule n'est pas douée de mobilité. Parfois, certains individus sont munis de deux flagelles locomoteurs. Il s'agit soit de cellules sexuées ou gamètes, qui assurent la reproduction, soit de zoospores qui interviennent dans la multiplication pour permettre la dissémination de l'espèce. La formation de ces deux types de cellules est assurée par la division d'une cellule initiale ou cellule mère.

En culture sur milieu gélosé, les cellules restent groupées en amas discoïdes.

Quelques différences distinguent les cellules fraîchement prélevées dans la piscine d'entreposage de celles cultivées pendant 1 mois dans le milieu BBM. Les premières possèdent de gros granules de réserve, sont pour la plupart agglomérées entre elles, maintenues par un mucilage constitué de polysaccharides, et la mobilité de leurs cellules flagellées est réduite. Lorsque les algues cultivées dans le milieu BBM sont remises en conditions de stress radioactif et nutritif, elles reprennent la morphologie de celles isolées à partir de l'eau de la piscine d'entreposage.

Les secondes sont plus pigmentées (vert vif), généralement bien individualisées et leurs cellules flagellées sont très actives lorsque la culture est régulièrement agitée.

Les modifications cytologiques et comportementales de cette micro-algue dans le milieu hostile à partir duquel elle a été isolée peuvent donc être perçues comme une adaptation aux conditions physico-chimiques particulières (présence de rayonnements ionisants, présence de métaux radioactifs induisant des stress oxydant et ionisant ; manque de sels minéraux, responsable d'un stress nutritif).

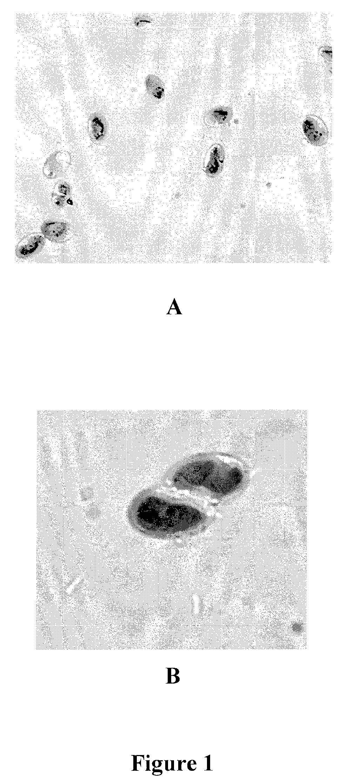

Les Figures 1A et 1B représentent des photographies de ces microalgues observées en microscopie photonique, un mois après l'introduction d'un échantillon provenant de la piscine du réacteur dans un milieu de culture BBM.

L'amidon, localisé dans le chloroplaste, peut être mis en évidence par une coloration au lugol (I₂ + IK = eau iodée). Une réponse positive à cette réaction (coloration brun jusqu'à bleu violacé) permet de classer cette micro-algue parmi les algues vertes (= Chlorophyta).

Le spectre d'absorption UV-visible de cet organisme montre la présence de chlorophylle a (pic d'absorption à 663 nm), chlorophylle b (pic d'absorption à 647 nm) et de carotène (pic d'absorption à 470 nm).

Il s'agit donc d'un taxon d'algues vertes, unicellulaire, eucaryote et dulçaquicole. Il appartient au règne des Protistes à affinité végétale, donc aux Protophytes. Il se situe dans l'embranchement des Chlorophytes ou Algues Vertes car il renferme comme pigments photosynthétiques les chlorophylles a et b et du carotène et ses réserves sont de l'amidon.

### Amplification et séquençage des gènes de l'ADN ribosomal

L'ADN total de la micro-algue A isolée comme décrit ci-dessus a été extrait par la méthode de Newman et al. (Genetics, 126 : 875-888, 1990).

La région du génome couvrant les gènes de l'ADN ribosomal ARNr18S-ITS1-ARNr5.8S-ITS2-ARNr26S (500 premières bases) a été amplifiée par PCR et séquencée.

Les amorces utilisées sont EAF3 : TCGACAATCTGGTTG ATCCTGCCAG (SEQ ID NO: 2) et ITS055R : CTCCTTGGTC CGTGTTTCAAGACGGG (SEQ ID NO: 3), classiquement utilisées pour amplifier les gènes des ARNr de micro-algues.

Les produits d'amplification obtenus en utilisant l'ADN isolé de deux cultures indépendantes sont de 4 kb (soit 1 kb de plus de ce qui est classiquement décrit pour les micro-algues). La séquence de ces produits d'amplification est représentée sur la Figure 2, ainsi que dans la liste des séquences en annexe sous le numéro SEQ ID NO: 1. Cette séquence contient 2 inserts d'environ 500 paires de bases qui sont indiquées en italique gras sur la Figure 2 et qui sont spécifiques à la micro-algue CCAP 216/25.

L'algorithme BLASTN (Altschul et al. Nucleic Acids Research, 25 : 3389-3402, 1997) a été utilisé pour rechercher, dans les bases de données, les séquences de gènes d'ARN ribosomal présentant le maximum d'identité avec la séquence SEQ ID NO: 1. Pour effectuer cette recherche, les deux séquences d'environ 500 paires de bases qui sont indiquées en italique gras sur la Figure 2 ont été retirées. Cette recherche a fait apparaître que les espèces caractérisées les plus proches de la micro-algue CCAP 216/25 appartiennent au genre *Coccomyxa.* La séquence SEQ ID NO: 1 contient toutefois 2 inserts spécifiques d'environ 500 paires de bases, qui ne se trouvent pas chez les algues du genre *Coccomyxa* caractérisées jusqu'à présent.

La comparaison des séquences correspondant à l'ARN de la petite sous-unité du ribosome (ARNr 18S) de la micro-algue CCAP 216/25, et des autres espèces de *Coccomyxa* répertoriées sur les bases de données (effectuée après avoir retiré les 2 inserts spécifiques de la séquence SEQ ID NO: 1) a été réalisée par alignement multiple des séquences au moyen du logiciel ClustalW2 2.0.12. L'alignement de séquences est présenté sur la Figure 3. Le Tableau II ci-dessous présente les résultats de cette comparaison de séquences. Les séquences correspondant aux ARNr 18S, qui sont comparées deux à deux, sont indiquées dans les colonnes SEQ ID A et SEQ ID B, par référence à leur numéro dans le listage de séquences en annexe.

**Tableau II**

| SEQ ID A | Nom | Longueur (nt) | SEQ ID B | Nom | Longueur (nt) | % d'identité |
|---|---|---|---|---|---|---|
| 4 | CCAP 216/25 | 2354 | 5 | Coccomyxa_peltigerae | 1783 | 97 |
| 4 | CCAP 216/25 | 2354 | 6 | Coccomyxa_chodatii | 1783 | 98 |
| 4 | CCAP 216/25 | 2354 | 7 | Coccomyxa_sp_flensburg | 1778 | 97 |
| 4 | CCAP 216/25 | 2354 | 8 | Coccomyxa_glaronensis | 1797 | 96 |
| 4 | CCAP 216/25 | 2354 | 9 | Coccomyxa_sp_CPCC | 1797 | 96 |
| 4 | CCAP 216/25 | 2354 | 10 | Chlamydomonas_sp | 1558 | 92 |
| | | | | | | |
| 5 | Coccomyxa_peltigerae | 1783 | 6 | Coccomyxa_chodatii | 1783 | 99 |
| 5 | Coccomyxa_peltigerae | 1783 | 7 | coccomyxa_sp_flensburg | 1778 | 96 |
| 5 | Coccemyxa_peltigerae | 1783 | 8 | Coccomyxa_glaronensis | 1797 | 96 |
| 5 | Coccomyxa_peltigerae | 1783 | 9 | Coccomyxa_sp_CPCC | 1797 | 96 |
| 5 | Coccomyxa_peltigerae | 1783 | 10 | Chlamydomonas_sp | 1558 | 92 |
| | | | | | | |
| 6 | Coccomyxa_chodatii | 1783 | 7 | Coccomyxa_sp_flensburg | 1778 | 96 |
| 6 | Coccomyxa_chodatii | 1783 | 8 | Coccomyxa_glaronensis | 1797 | 96 |
| 6 | Coccomyxa_chodatii | 1783 | 9 | Coccomyxa_sp_CPCC | 1797 | 97 |
| 6 | Coccomyxa_chodatii | 1783 | 10 | Chlamydomonas_sp | 1558 | 92 |
| | | | | | | |
| 7 | Coccomyxa_sp_flensburg | 1778 | 8 | Coccomyxa_glaronensis | 1797 | 99 |
| 7 | Coccomyxa_sp_flensburg | 1778 | 9 | Coccomyxa_sp_CPCC | 1797 | 99 |
| 7 | Coccomyxa_sp_flensburg | 1778 | 10 | Chlamydomonas_sp | 1558 | 91 |
| | | | | | | |
| 8 | Coccomyxa_glaronensis | 1797 | 9 | Coccomyxa_sp_CPCC | 1797 | 99 |
| 8 | Coccomyxa_glaronensis | 1797 | 10 | Chlamydomonas_sp | 1558 | 91 |
| 9 | Coccomyxa_sp_CPCC | 1797 | 10 | Chlamydomonas_sp | 1558 | 91 |

Cette comparaison de séquences montre que les espèces les plus proches de la souche CCAP 216/25 sont *Coccomyxa chodatii* souche SAG 216-2 (SEQ ID NO: 6), *Coccomyxa peltigerae* souche SAG 216-5 (SEQ ID NO: 5), *Coccomyxa* sp. Flensburg fjord 2 (EU127471), *Coccomyxa glaronensis* souche CCALA 306 (AM167525) et *Coccomyxa* sp. souche CPCC 508 (AM981206) avec respectivement 98%, 97%, 97%, 96% et 96% d'identité de séquence. Ces forts scores d'identité obtenus pour la souche CCAP 216/25 par rapport au genre *Coccomyxa* sont voisins de ceux obtenus après comparaison des séquences des *Coccomyxa* entre elles et éloignés du score obtenu pour la comparaison de séquence avec une micro-algue unicellulaire appartenant à un autre genre (*Chlamydomonas* sp. CCMP681 (EF106784) (cf. Figure 3). Cela indique l'appartenance de la souche CCAP 216/25 au genre *Coccomyxa.*

Par ailleurs, la comparaison des séquences de la région des ITS de la souche CCAP 216/25 avec celles des autres *Coccomyxae* a également été réalisée. Le Tableau III ci-dessous présente les résultats de cette comparaison de séquences. Les séquences des régions ITS1-ARNr5.8S-ITS2 qui sont comparées deux à deux sont indiquées dans les colonnes I et II. Les séquences des souches *Coccomyxa actinabiotis* (CCAP 216/25), *Coccomyxa chodatii* SAG 216-2 et *Coccomyxa peltigerae* SAG 216-5 sont indiquées par référence à leur numéro dans le listage de séquences en annexe, à savoir respectivement SEQ ID NO 11, 12 et 13. Pour les souches suivantes, dont les séquences des régions ITS1-ARNr5.8S-ITS2 sont accessibles sur la base de données GenBank, les numéros d'accès correspondants sont également indiqués dans le Tableau III :

| | |
|---|---|
| - AY293964: | *Coccomyxa peltigerae var. variolosae* |
| - AY293965: | *Coccomyxa solarinae var. croceae* |
| - AY293966: | *Coccomyxa solarinae var. bisporae* |
| - AY293967: | *Coccomyxa solarinae var*. *saccatae* |
| - AY293968: | *Coccomyxa chodatii* |
| - AY328522: | *Coecomyxa peltigerae strain SAG 216-5* |
| - AY328523: | *Coccomyxa subellipsoidea strain SAG 216-13* |
| - AY328524: | *Coccomyxa rayssiae strain SANG 216-8* |
| - U66945 : | *Chlamydomonas callosa* |
| - U66956 : | *Dunaliella tertiolecta* |
| - AF376740: | *Pandorina morum* |

**Tableau III**

| I | Longueur (nt) | II | Longueur (nt) | % d'identité |
|---|---|---|---|---|
| ====================================================================== | | | | |
| SEQ ID 11 | 761 | SEQ ID 12 | 617 | 81 |
| SEQ ID 11 | 761 | SEQ ID 13 | 708 | 79 |
| SEQ ID 11 | 761 | AY293964 | 610 | 78 |
| SEQ ID 11 | 761 | AY293965 | 650 | 73 |
| SEQ ID 11 | 761 | AY293966 | 651 | 73 |
| SEQ ID 11 | 761 | AY293967 | 651 | 73 |
| SEQ ID 11 | 761 | AY293968 | 619 | 76 |
| SEQ ID 11 | 761 | AY328522 | 725 | 73 |
| SEQ ID 11 | 761 | AY328523 | 702 | 75 |
| SEQ ID 11 | 761 | AY328524 | 696 | 76 |
| SEQ ID 11 | 761 | U66945 | 625 | 43 |
| SEQ ID 11 | 761 | U66956 | 598 | 45 |
| SEQ ID 11 | 761 | AF376740 | 626 | 27 |
| | | | | |
| SEQ ID 12 | 617 | SEQ ID 12 | 708 | 87 |
| SEQ ID 12 | 617 | AY293964 | 610 | 83 |
| SEQ ID 12 | 617 | AY293965 | 650 | 82 |
| SEQ ID 12 | 617 | AY293966 | 651 | 83 |
| SEQ ID 12 | 617 | AY293967 | 651 | 83 |
| SEQ ID 12 | 617 | AY293968 | 619 | 92 |
| SEQ ID 12 | 617 | AY328522 | 725 | 84 |
| SEQ ID 12 | 617 | AY328523 | 702 | 80 |
| SEQ ID 12 | 617 | AY328524 | 696 | 94 |
| SEQ ID 12 | 617 | U66945 | 625 | 38 |
| SEQ ID 12 | 617 | U66956 | 598 | 31 |
| SEQ ID 12 | 617 | AF376740 | 626 | 26 |
| | | | | |
| SEQ ID 13 | 708 | AY293964 | 610 | 97 |
| SEQ ID 13 | 708 | AY293965 | 650 | 99 |
| SEQ ID 13 | 708 | AY293966 | 651 | 99 |
| SEQ ID 13 | 708 | AY293967 | 651 | 99 |
| SEQ ID 13 | 708 | AY293968 | 619 | 88 |
| SEQ ID 13 | 708 | AY328522 | 725 | 94 |
| SEQ ID 13 | 708 | AY328523 | 702 | 80 |
| SEQ ID 13 | 708 | AY328524 | 696 | 86 |
| SEQ ID 13 | 708 | U66945 | 625 | 37 |
| SEQ ID 13 | 708 | U66956 | 598 | 51 |
| SEQ ID 13 | 708 | AF376740 | 626 | 34 |
| | | | | |
| AY293964 | 610 | AY293965 | 650 | 97 |
| AY293964 | 610 | AY293966 | 651 | 97 |
| AY293964 | 610 | AY293967 | 651 | 97 |
| AY293964 | 610 | AY293968 | 619 | 83 |
| AY293964 | 610 | AY328522 | 725 | 96 |
| AY293964 | 610 | AY328523 | 702 | 78 |
| AY293964 | 610 | AY328524 | 696 | 85 |
| AY293964 | 610 | U66945 | 625 | 41 |
| AY293964 | 610 | U66956 | 598 | 50 |
| AY293964 | 610 | AF376740 | 626 | 48 |
| | | | | |
| AY293965 | 650 | AY293966 | 651 | 99 |
| AY293965 | 650 | AY293967 | 651 | 99 |
| AY293965 | 650 | AY293968 | 619 | 88 |
| AY293965 | 650 | AY328522 | 725 | 98 |
| AY293965 | 650 | AY328523 | 702 | 80 |
| AY293965 | 650 | AY328524 | 696 | 85 |
| AY293965 | 650 | U66945 | 625 | 37 |
| AY293965 | 650 | U66956 | 598 | 45 |
| AY293965 | 650 | AF376740 | 626 | 34 |
| | | | | |
| AY293966 | 651 | AY293967 | 651 | 99 |
| AY293966 | 651 | AY293968 | 619 | 88 |
| AY293966 | 651 | AY328522 | 725 | 98 |
| AY293966 | 651 | AY328523 | 702 | 79 |
| AY293966 | 651 | AY328524 | 696 | 85 |
| AY293966 | 651 | U66945 | 625 | 37 |
| AY293966 | 651 | U66956 | 598 | 51 |
| AY293966 | 651 | A376740 | 626 | 34 |
| | | | | |
| AY293967 | 651 | AY293968 | 619 | 88 |
| AY293967 | 651 | AY328522 | 725 | 98 |
| AY293967 | 651 | AY328523 | 702 | 79 |
| AY293967 | 651 | AY328524 | 696 | 85 |
| AY293967 | 651 | U66945 | 625 | 37 |
| AY293967 | 651 | U66956 | 598 | 51 |
| AY293967 | 651 | AF376740 | 626 | 34 |
| | | | | |
| AY293968 | 619 | AY328522 | 725 | 87 |
| AY293968 | 619 | AY328523 | 702 | 82 |
| AY293968 | 619 | AY328524 | 696 | 96 |
| Y293968 | 619 | U66945 | 625 | 37 |
| AY293968 | 619 | U66956 | 598 | 40 |
| AY293968 | 619 | AF376740 | 626 | 37 |
| | | | | |
| AY328522 | 725 | AY328523 | 702 | 83 |
| AY328522 | 725 | AY328524 | 696 | 88 |
| AY328522 | 725 | U66945 | 625 | 37 |
| AY328522 | 725 | U66956 | 598 | 51 |
| AY328522 | 725 | AF376740 | 626 | 34 |
| | | | | |
| AY328523 | 702 | AY328524 | 696 | 84 |
| AY328523 | 702 | U66945 | 625 | 38 |
| AY328523 | 702 | U66956 | 598 | 36 |
| AY328523 | 702 | AF376740 | 626 | 35 |
| | | | | |
| AY328524 | 696 | U66945 | 625 | 37 |
| AY328524 | 696 | U66956 | 598 | 31 |
| AY328524 | 696 | AF376740 | 626 | 26 |
| | | | | |
| U66945 | 625 | U66956 | 598 | 58 |
| U66945 | 625 | AF376740 | 626 | 59 |
| | | | | |
| U66956 | 598 | AF376740 | 626 | 51 |

Cette comparaison de séquences montre une identité de 73% à 81% de la séquence de la souche CCAP 216/25 par rapport aux autres souches *Coccomyxae* référencées, du même ordre que les scores obtenus pour la comparaison de la région des ITS des *Coccomyxae* référencées entre eux (78% à 99%), et très éloigné de celui obtenu pour la comparaison avec d'autres genres (de l'ordre de 30 à 50%).

D'autre part, il s'avère que la séquence correspondant à l'ARNr 18S de la souche CCAP 216/25 est différente de celle de toutes les *Coccomyxae* référencées jusqu'à présent, notamment en ce qu'elle possède deux insertions d'environ 500 paires de bases.

Ces deux derniers points indiquent qu'il s'agit d'une espèce de *Coccomyxae* différente de toutes celles référencées à ce jour.

Ces résultats indiquent donc que la micro-algue isolée appartient au genre *Coccomyxa,* mais que son ADN diffère suffisamment de celui des autres espèces de *Coccomyxae* répertoriées dans les banques de données, notamment par ses deux insertions dans l'ADN ARNr 18S et des ITS1 et ITS2 pour que l'on considère qu'il s'agit d'une nouvelle espèce, qui sera dénommée ici *Coccomyxa actinabiotis.*

### Résistance aux stress abiotiques

*Coccomyxa actinabiotis* est résistante à la dessiccation, au froid, à l'obscurité pendant 48h.

Elle est également résistante à l'eau lourde pure pendant 48 h (la respiration et la photosynthèse d'algues exposées pendant 24 h et 48 h à D₂O 50%, 80% et 100%), à l'obscurité, et leur récupération après 24 h dans un milieu normal ainsi que la respiration et la photosynthèse d'algues exposées pendant 24 h à la lumière dans 50% D₂O montre que l'eau lourde n'a pas dans ces conditions d'effet sur ces paramètres physiologiques).

Cette résistance à l'eau lourde permet de proposer l'utilisation de *Coccomyxa actinabiotis* pour la synthèse de composés deutérés.

*Coccomyxa actinabiotis* est également résistante à l'acétone, à l'alcool, aux acides, aux ultrasons.

La résistance aux rayonnements ionisants des algues a été testée en les exposant à différentes doses de rayonnements gamma issus d'éléments de combustible usés en cours de décroissance. La mortalité après irradiation a été déterminée par colorant vital (rouge neutre).

*Coccomyxa actinabiotis* est résistante à long terme (plus d'une dizaine d'années) à une irradiation modérée (150 µGy/h).

Elle résiste à une exposition de plusieurs jours à un débit de dose de 300 Gy/h et pendant quelques heures à un débit de dose de 3000 Gy/h.

Elle résiste également à une exposition pendant 20 h à un débit de dose de 1 kGy/h.

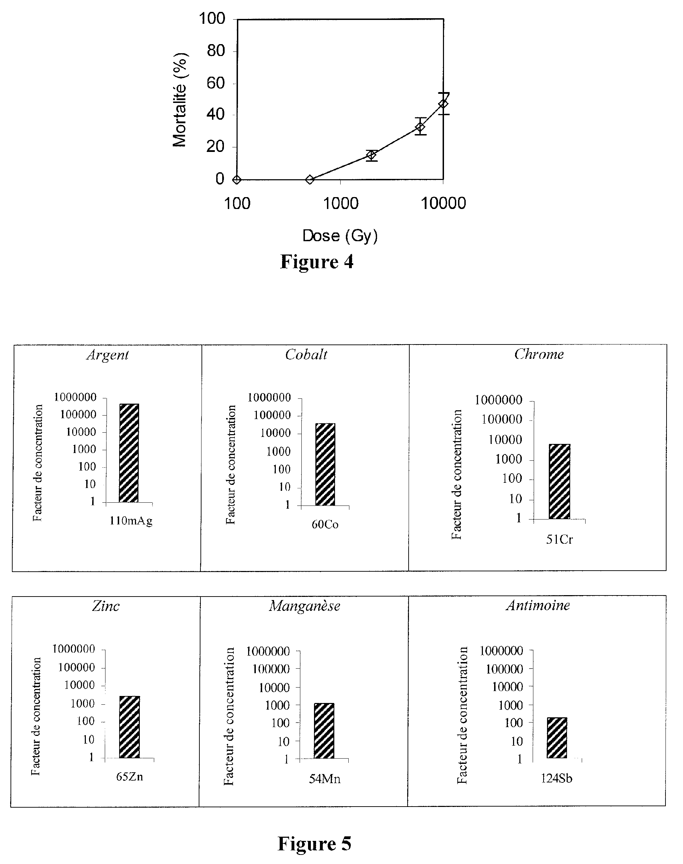

La Figure 4 montre le pourcentage de mortalité de *Coccomyxa actinabiotis* en fonction de la dose d'irradiation. La dose de rayonnements ionisants qui provoque la mort de la moitié de la population est de plus de 10 kGy (45% de mortalité à 10 kGy).

*Coccomyxa actinabiotis* résiste également remarquablement à une exposition à 20 kGy (80% ± 5% de mortalité, soit 20% ± 5% de survivantes). Dans le cas de cette forte irradiation à 20 kGy, elle recolonise le milieu en quelques semaines. En 4 semaines, la culture a récupéré le tiers de son activité photosynthétique initiale avant irradiation, et elle récupère en 15 jours une densité de population égale à celle d'échantillons non irradiés ou faiblement irradiés.

### EXEMPLE 2 : CONCENTRATION DE MÉTAUX RADIOACTIFS PAR COCCOMYXA ACTINABIOTIS

### Fixation et concentration des métaux

Les micro-algues fraîchement prélevées dans la piscine d'entreposage (environ 10 milligrammes de masse fraîche) ont été mises en présence, sous éclairement (200 lux), pendant 1 an, d'eau déminéralisée, renouvelée régulièrement, de pH 5,5, de conductivité 1,2 µS/cm, contenant par exemple 400 Bq/l de ⁶⁰Co, 1100 Bq/l de ^{110m}Ag, 1700 Bq/l de ¹²⁴Sb, 5000 Bq/l de ⁵¹Cr, 400 Bq/l de ⁶⁵Zn, 300 Bq/l de ⁵⁴Mn. L'eau est radioactive essentiellement du fait de la présence de tritium (environ 300000 Bq/l) et de ¹⁴C (environ 20000 Bq/l). Cette composition est similaire à celle de l'eau des piscines d'entreposage de combustibles usés. Les algues se sont multipliées lentement pendant cette mise en contact.

Les concentrations des radionucléides ont été mesurées à l'équilibre dans les eaux et dans les algues par spectrométrie γ.

Les facteurs de concentration de chaque métal (^{110m}Ag, ⁶⁰Co, ⁵¹Cr, ⁶⁵Zn, ⁵⁴Mn et ¹²⁴Sb), définis comme le rapport de la concentration du métal adsorbé ou absorbé dans les micro-algues (en atomes/g de matière fraîche) à la concentration du métal dans l'eau (atomes/ml), ont ainsi été obtenus.

Les résultats sont illustrés par la Figure 5.

Ces résultats montrent que les micro-algues concentrent très fortement l'argent et le cobalt (facteur de concentration = 450000 et 40000, respectivement), ainsi que le chrome dans une moindre mesure (facteur de concentration = 6000). Les éléments les moins concentrés ont aussi un facteur de concentration significatif (facteur de concentration = 6000, 3000, 1200 et 200 pour le chrome, le zinc, le manganèse et l'antimoine, respectivement).

Une autre expérience a été réalisée en mettant en présence 250 mg de masse fraîche de micro-algues provenant de cultures en milieu BBM avec 100 ml d'eau déminéralisée de composition similaire à celle de l'eau des piscines d'entreposage de combustibles usés à savoir pH 5,5, conductivité 1,2 µS/cm, contenant 280 Bq/l de ⁶⁰Co, 530 Bq/l de ⁵⁸Co, 66 Bq/l de ^{110m}Ag, 1460 Bq/l de ¹²⁴Sb, 1180 Bq/l de ⁵¹Cr, 120 Bq/l de ⁶⁵Zn. Bq/l de ⁵⁴Mn ainsi que du tritium (260000 Bq/l) et du ¹⁴C (10000 Bq/l). Après une journée de culture, le pourcentage de chaque isotope fixé est de 100%, 100%, 94%, 94%, 90%, 48% et 30% pour ^{110m}Ag, ⁶⁵Zn, ⁶⁰Co, ⁵⁸Co, ⁵⁴Mn, ⁵¹Cr et ¹²⁴Sb, respectivement.

### Extraction des métaux à partir des micro-algues

Des expériences de solubilisation des métaux fixés par les algues ont été effectuées, en incubant ces algues (70 mg de masse fraîche) dans 5 ml d'eau déminéralisée, ou d'eau déminéralisée additionnée d'HCl à différentes concentrations. En milieu neutre, les métaux ne sont pas extraits.

Les cations les plus retenus sont ⁶⁰Co, ^{108m}Ag et ^{110m}Ag. Ils restent fixés à la cellule pour des faibles concentrations en HCl (pH 4,5 à 2,5). Ils commencent à être libérés de façon exponentielle en dessous d'un pH seuil de 2,5, Co étant le plus solidement fixé puisqu'il est au maximum extrait à 35 % au bout de 6 jours d'exposition à pH 0,5, alors que Ag l'est à 90 % dans les mêmes conditions. A pH 1,5, 10% de Ag et 2% de Co sont extraits après 6 jours d'exposition. Pour ⁵¹Cr, il n'y a pas de pH seuil, il est extrait dès pH = 4,5 de façon linéaire jusqu'à un maximum de 25 % à pH = 0,5 après 6 jours d'exposition.

D'autres expériences ont été effectuées en présence d'une solution 0,1 M d'EDTA de pH 4,5. Dans ces conditions, 35% du ⁵¹Cr contenu dans les algues, mais seulement 1% du ⁶⁰Co et moins de 0,5% de ^{108m}Ag et ^{110m}Ag, sont extraits après 2 jours d'exposition.

Ces expériences montrent que le Cr reste majoritairement en surface, tandis que le Co est probablement majoritairement séquestré à l'intérieur des cellules et Ag se répartit entre la surface et l'intérieur des cellules.

### EXEMPLE 3 : CINÉTIQUE DE FIXATION DES MÉTAUX, RÉSISTANCE À LEUR TOXICITÉ ET QUANTITES FIXEES

### Cinétiques de fixation et d'incorporation de Ag et Co

Les cinétiques de fixation des métaux par les micro-algues *Coccomyxa actinabiotis,* ainsi que les quantités ad-absorbées, ont été déterminées, dans le cas d'un cation monovalent, Ag⁺, et d'un cation divalent, Co²⁺,

### Argent

Deux séries d'expériences ont été menées ; l'une avec des algues présentant une gangue de mucilage dont l'épaisseur, évaluée par microscopie optique, est de l'ordre de 1 µm ou plus (« algues avec mucilage »), l'autre avec des algues cultivées en milieu BBM et ne présentant pas ou peu de mucilage (« algues sans mucilage »).

Dans des erlenmeyers on introduit 20 mg d'algues (masse fraîche compactée) et 90 ml de solution d'Ag⁺ à différentes concentrations. Les 20 mg d'algues sont préparés comme suit : prélèvement dans un ballon de culture, centrifugation 20 minutes à 3000 rpm, lavage deux fois dans de l'eau déminéralisée (mise en suspension, centrifugation). Le milieu de culture est ainsi éliminé et ne risque pas de complexer l'argent.

Les algues sans mucilage sont mises en contact avec des solutions contenant 0,11 mg/L et 5,5 mg/L d'Ag⁺.

Les algues avec mucilage sont mises en contact avec des solutions contenant 22 µg/L, 1,1 mg/L et 55 mg/L d'Ag⁺.

Les erlenmeyers sont placés sur des agitateurs éclairés et des prélèvements de 5 ml sont effectués pour différents temps d'exposition afin de suivre les cinétiques de fixation pour chacune des concentrations initiales en argent. Les prélèvements sont centrifugés 10 minutes à 4500 rpm pour séparer les algues qui ont concentré le métal. La teneur en métal est ensuite analysée dans le culot (algues) et dans le surnageant.

Les résultats sont illustrés par la Figure 6 pour les algues sans mucilage, et par la Figure 7 pour les algues avec mucilage.

On observe, pour les algues avec ou sans mucilage, une cinétique d'absorption de l'argent très rapide dans les premières heures, suivie d'un plateau.

Au plateau, les algues sans mucilage fixent la totalité de l'argent si sa concentration initiale est de 0,11 mg/l et en fixent 75% si sa concentration initiale est de 5,5 mg/l.

Les algues avec mucilage fixent la totalité de l'argent si sa concentration initiale est de 1,1 mg/l et en fixent 20% si sa concentration initiale est de 55 mg/l.

L'accumulation rapide de l'argent au niveau des algues peut s'expliquer par deux phénomènes. D'une part par une chélation à l'extérieur des micro-algues par le mucilage abondant, constitué de polymères de sucres et dérivés, qui a la propriété de chélater les cations. D'autre part par une incorporation active et/ou passive de l'argent à l'intérieur des algues par des canaux ou des transporteurs. Les nombreux canaux ioniques transmembranaires par exemple assurent un transport passif non sélectif des ions Na⁺, K⁺ ou d'éventuels métaux monovalents tels Ag⁺ vers le cytoplasme, d'autant plus facilement que le gradient de concentration est élevé.

Ainsi la concentration de l'argent par *Coccomyxa* correspond à une adsorption sur le mucilage et les composés pariétaux à laquelle s'ajoute une absorption intracellulaire via des canaux ioniques transmembranaires et des transporteurs actifs.

### Cobalt

Des expériences ont été effectuées avec des algues ne présentant pas ou peu de mucilage, selon le même protocole que celui décrit ci-dessus pour l'argent. Les algues ont été exposées à des concentrations initiales de cobalt de 19 µg/L, 0,94 mg/L et 4,8 mg/L.

Les résultats sont représentés sur la Figure 8 (A-C).

Une adsorption, éventuellement couplée à une absorption, initiale rapide est observée. Elle est cependant moins marquée pour le cobalt que pour l'argent. Puis l'absorption se poursuit progressivement sur plusieurs jours. La concentration de cobalt intracellulaire ne plafonne pas mais continue d'augmenter plus d'une semaine après le début de l'exposition.

Le mécanisme d'absorption du cobalt apparaît donc différent de celui de l'argent. Les ions Co²⁺ divalents ne peuvent en effet emprunter les mêmes canaux ioniques que les ions Ag⁺ en raison de leur rayon ionique plus important. Il est vraisemblable que des co-transporteurs, chargés de l'absorption de métaux divalents indispensables à la croissance cellulaire (Fe²⁺, Mg²⁺, ...) ou des transporteurs actifs, tels les pompes ATP-dépendantes ABC connues comme transporteurs transmembranaire du cadmium Cd²⁺ ou des ATPases à Zn²⁺, assurent l'absorption du cobalt. Ce transport actif expliquerait une absorption modérée mais durable.

### Concentrations maximales de métaux tolérées

Les doses limites supportées par *Coccomyxa actinabiotis* ont été recherchées par exposition des algues à l'argent ou au cobalt durant une semaine (20 mg de micro-algues (masse fraîche) dans 100 ml d'eau désionisée contenant les ions Ag ou Co à différentes concentrations).

Les algues sans mucilage sont moins résistantes à la toxicité métallique de l'argent que les algues avec mucilage. Le mucilage pourrait éviter un afflux rapide et intense de métal dans le cytoplasme au début de l'exposition. En constituant une barrière fixatrice des ions, il limite la diffusion vers les canaux transmembranaires et l'importation rapide et massive de métaux toxiques. Ces métaux seraient alors absorbés de manière modérée et progressive et pourraient être stockés sous des formes non agressives pour la cellule. Des *Coccomyxae actinabiotis* sans mucilage ne survivent pas à des concentrations d'argent supérieures à 250 µg/L alors que les *Coccomyxa actinabiotis* avec mucilage supportent des concentrations extracellulaires d'argent d'au moins 50 mg/L.

Le seuil d'intolérance au cobalt est supérieur à des concentrations de Co²⁺ de 50 mg/l et 800 mg/l pour *Coccomyxa actinabiotis* sans mucilage et avec mucilage, respectivement. Aucun effet notable n'a été constaté sur les micro-algues cultivées pendant une semaine dans ces conditions.

### Concentrations maximales de métaux fixées

Les algues peuvent fixer des quantités d'Ag et de Co beaucoup plus importantes que ce qui pourrait être attendu par une fixation passive, en particulier du fait de leur synthèse de mucilage abondant et de mécanismes actifs d'incorporation et de détoxification.

De manière générale, les métaux sont toxiques pour les organismes.

L'argent est généralement toxique pour les organismes à 10⁻⁷ - 10⁻⁵ mol/l (Ratte HT, Environ. Toxicol. Chem. 18 : 89-108, 1999).

Des organismes radiorésistants comme *Deinococcus radiodurans* ne se développent pas en présence de cobalt à 10⁻⁴ mol/l (John *et al.,* 2000, ci-dessus). Or la micro-algue *Coccomyxa actinabiotis* croît en présence de 10⁻⁴ mol/l de cobalt.

Des algues *Coccomyxa actinabiotis* mises en contact dans un milieu de culture BBM dilué 10 fois avec un équivalent de 6,7.10⁻⁴ mole d'argent par gramme d'algue fixent jusqu'à 43,7 mg d'argent par gramme de masse fraîche soit environ 450 mg/g de masse sèche.

Cette quantité d'argent fixée est très supérieure à celles rapportées dans la littérature. La littérature rapporte des concentrations d'argent dans les plantes terrestres comprises entre 0,01 et 150 µg d'argent/g de masse sèche, soit environ 0,001 à 15 µg d'argent/g de masse fraîche, et des concentrations d'argent dans les algues comprises entre 3 µg/g et 7 mg/g de masse sèche, soit environ 0,3 µg à 0,7 mg d'argent/g de masse fraîche. La quantité d'argent fixée par *Coccomyxa actinabiotis* est même plus importante que la quantité d'argent accumulable par des bactéries qui présentent une tolérance à l'argent extrêmement élevée : des bactéries hyperaccumulatrices d'argent accumulent jusqu'à 300 mg d'argent/g de masse sèche (Ratte, 1999, ci-dessus ; Charley et al., Arch. Microbiol. 123 : 239-244, 1979). D'autre part, les organismes les plus accumulateurs d'argent ne sont pas radiorésistants.

Des algues *Coccomyxa actinabiotis* mises en contact pendant 45 jours avec un équivalent de 3,8.10⁻⁴ mole de cobalt par gramme d'algue dans un milieu nutritif BBM dilué 10 fois fixent 12,1 mg de métal par gramme de biomasse fraîche. En 6 jours, des algues mises en contact avec un équivalent de 1,0.10⁻³ mole de cobalt par gramme d'algue dans un milieu nutritif BBM dilué 10 fois fixent 1,53 mg de métal par gramme de biomasse fraîche. Ces valeurs sont largement supérieures à celles rapportées dans la littérature. La concentration moyenne de cobalt dans les plantes varie entre 0,1 et 115 µg/g de masse sèche, soit environ 0,01 à 11 µg/g de masse fraîche, et des plantes hyperaccumulatrices de cobalt en fixent jusqu'à 4,3 mg/g de masse sèche (Bresson et al., Toxicologie nucléaire, environnementale et humaine, Lavoisier éd., 2009, Editions TEC et DOC. Chap. 29 Cobalt, p. 553-573), soit environ 0,4 mg/g de masse fraîche. Les organismes les plus accumulateurs de cobalt cités dans la littérature ne sont pas radiorésistants.

### EXEMPLE 4 : FIXATION DU CARBONE 14

Le carbone 14 constitue, avec le tritium, le principal polluant radioactif rejeté par les installations nucléaires dans l'environnement (8 à 25 GBq de ¹⁴C/an dans les rejets liquides des installations EDF). Les rejets en ¹⁴C ne sont réglementés que depuis 2008. Pour le moment, il n'existe pas de traitement spécifique du ¹⁴C. La réglementation en matière de ¹⁴C dans les rejets radioactifs liquides varie d'une installation à l'autre. Le seuil est fixé à 150-400 GBq pour une centrale EDF (arrêté 2008). Le ¹⁴C est rejeté dans les effluents liquides essentiellement sous forme de ¹⁴CO₂, carbonate (¹⁴CO₃²⁻ ou H¹⁴CO₃⁻), ¹⁴CO et un faible pourcentage sous forme organique non identifiée. Comme tous les végétaux, la micro-algue verte *Coccomyxa* est capable d'incorporer du carbone sous forme de ¹²CO₂ via la photosynthèse. La discrimination isotopique du ¹⁴C par rapport au ¹²C est négligeable dans les processus biologiques. Mais contrairement à ce qui pourrait en être déduit, la fixation du ¹⁴C par la micro-algue *Coccomyxa actinabiotis* n'a rien d'évident. Par exemple, l'algue ne fixe pas le ¹⁴C à pH 8 mais le fixe à pH 7.

D'autre part, la fixation du ¹⁴C via des processus biologiques tels que la photosynthèse ne peut être réalisée qu'avec de la biomasse photosynthétique et vivante.

L'impact du ¹⁴C sur la physiologie des micro-algues et les paramètres permettant de réaliser et d'optimiser son accumulation par ces micro-organismes ont été déterminés.

Le ¹⁴C est dosé dans les algues et dans les eaux par scintillation liquide. Les micro-algues sont mises en contact avec du ¹⁴C sous forme de carbonate CO₃²⁻ en équilibre acido-basique avec HCO₃⁻ et CO₂ (pKa de 6,4 et 10,3) ainsi que sous forme d'acétate.

### Impact du ¹⁴C sur la physiologie des micro-algues

L'impact de la présence de ¹⁴C sur la physiologie des algues a été évalué en suivant la croissance des cellules. La présence de ¹⁴C seul, jusqu'à 20000 Bq/l, n'a pas d'impact sur la croissance, comparée à celle d'une culture non exposée au ¹⁴C.

Des cellules mises en culture dans une eau de piscine d'entreposage de combustibles nucléaires usés, contenant des émetteurs gamma et enrichie avec 300000 Bq/l de ¹⁴C, ont également vu leur population croître pendant 8 jours.

### Paramètres influençant l'incorporation du ¹⁴C par les algues

### Forme chimique du ¹⁴C

Les algues incorporent la forme minérale gaz carbonique en équilibre avec l'hydrogénocarbonate tout comme la forme organique acétate. Le CO₂ est incorporé dans les cellules via la photosynthèse (6 CO₂ + 12 H₂O + hv → C₆H₁₂O₆ + 6 O₂ + H₂O). L'acétate est utilisé par le métabolisme sous forme d'acétylcoenzymeA et notamment dans le cycle énergétique principal de la cellule (cycle de Krebs).

### pH

L'influence du pH sur l'incorporation du ¹⁴C sous forme d'hydrogénocarbonate a été étudiée dans la gamme 6,9 à 8,5. Le pH optimum est de 6,9 et permet l'incorporation de 80 à 90% du ¹⁴C présent dans le milieu lorsque les récipients dans lesquels s'effectue la mise en contact sont fermés hermétiquement. A ce pH, 25% du ¹⁴C est sous forme de CO₂ dissous. Il est nécessaire de fermer les récipients pour éviter le dégazage rapide du ¹⁴CO₂ en équilibre avec H¹⁴CO₃⁻.

La quantité de ¹⁴C incorporée par les algues est faible à pH 7,5 (quelques %) et nulle à pH 8,5.

### Conditions initiales de culture des micro-algues

L'état physiologique des cellules au moment de la mise en contact avec le ¹⁴C a une influence sur la vitesse d'incorporation du ¹⁴C. Les micro-algues incorporent beaucoup plus rapidement le ¹⁴C lorsqu'elles ont été préalablement cultivées dans un milieu plus riche en nutriments (milieu BBM dilué deux fois préférable à un milieu BBM dilué dix fois). Ainsi, le temps optimal de mise en contact des algues avec un milieu contenant 20000 Bq/l de ¹⁴C est de 50 h si la culture est issue d'un milieu BBM dilué dix fois tandis qu'il est réduit à 3 h si la culture est issue d'un milieu BBM dilué deux fois.

### Densité des algues

La quantité de ¹⁴C incorporée en une durée donnée augmente avec la densité des algues dans la gamme 5.10⁶ à 15.10⁶ cellules/ml. A titre d'exemple, il faut 50 h à une population algale de densité cellulaire initiale 5.10⁶ cellules/ml pour incorporer 70 % de la quantité de ¹⁴C présente dans le milieu (20000 Bq/l) alors qu'il suffit de 7,5 h à une population algale de densité cellulaire initiale 15.10⁶ cellules/ml pour en incorporer 90 %.

### Intensité de l'éclairement

Les expériences d'incorporation du ¹⁴C ont été réalisées sous faible éclairement (50 à 70 µmol de photons/m²/s). Or l'activité photosynthétique augmente avec l'éclairement jusqu'à atteindre un maximum à partir de 500 µmol de photons/m²/s. La mesure de l'activité photosynthétique maximale des algues, réalisée avec un éclairement de plus de 500 µmol de photons/m²/s, montre que les algues sont capables de consommer beaucoup plus de ¹²CO₂ sous plus fort éclairement (au moins 30 fois plus qu'aux intensités lumineuses auxquelles ont été faites les expériences d'incorporation du ¹⁴C).

### Concentration en ¹⁴C

Quelle que soit la concentration initiale du ¹⁴C dans la gamme 2000 à 20000 Bq/l (qui correspond aux concentrations présentes dans les eaux des piscines de certaines installations nucléaires), à pH 6,9, les algues incorporent rapidement et fortement le ¹⁴C. La vitesse maximale d'incorporation du ¹⁴C par une population d'algues issue d'une culture en milieu BBM dilué deux fois est de 20 Bq/h/10⁶ cellules pour une concentration de ¹⁴C de 6000 Bq/l et de 60 Bq/h/10⁶ cellules pour une concentrations de ¹⁴C de 20000 Bq/l. Les quantités incorporées sont de l'ordre de 80-90%.

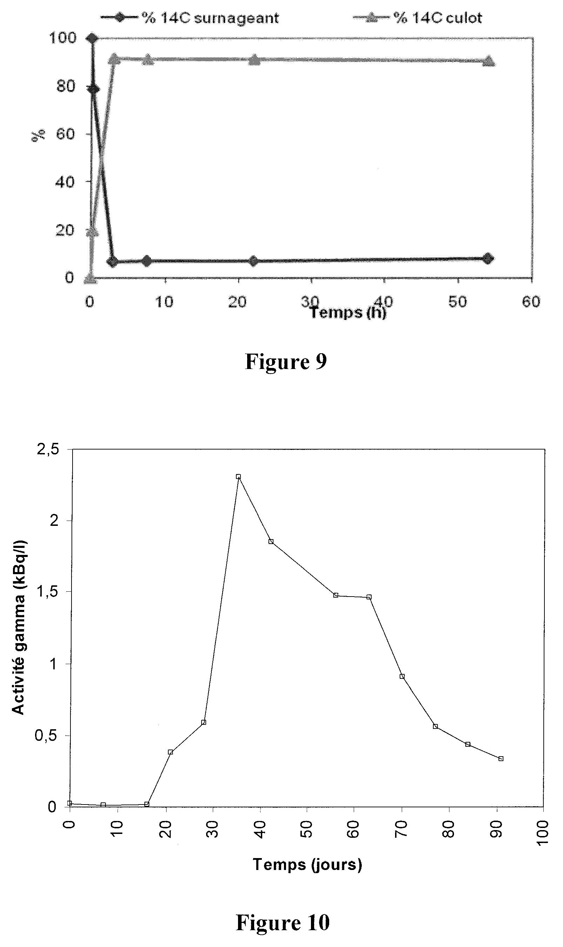

L'incorporation du ¹⁴C sous forme d'hydrogénocarbonate, à pH 6,9, par une culture de *Coccomyxa actinabiotis* issue d'un milieu BBM dilué deux fois, à la concentration initiale de 5.10⁶ cellules/ml, éclairée par 70 µmol de photons/m²/s, a été mesurée. La concentration en ¹⁴C est de 6000 Bq/l. Après centrifugation de prélèvements aux différents temps, la teneur en ¹⁴C est analysée dans le culot (algues) et dans le surnageant. Les résultats sont illustrés par la Figure 9.

### Conditions optimales de fonctionnement pour l'épuration du ¹⁴C

- pH 6,9
- cellules initialement cultivées dans un milieu riche en nutriments (BBM dilué deux fois)
- densité cellulaire de 15.10⁶ cellules/ml
- éclairage intense (500 µmol de photons/m²/s)
- durée de mise en contact : quelques heures

### Performances

### • 90% de decontamination

*Coccomyxae actinabiotis* peut donc être utilisée pour décontaminer le carbone 14 contenu dans des eaux issues d'installations nucléaires : 90% de décontamination en 3 à 7 heures, dans des conditions optimisées.

### EXEMPLE 5 : FIXATION DE L'URANIUM

Les algues selon l'invention peuvent aussi être utilisées pour capter l'uranium et les transuraniens.

Des micro-algues *Coccomyxae actinabiotis* récoltées à partir d'un ballon de culture et lavées trois fois avec de l'eau Milli-Q pour éliminer le milieu de culture ont été mises en contact, sous agitation et à une concentration équivalente à 60 mg d'algue en masse fraîche/100 ml de solution, avec une solution de nitrate d'uranyle de concentration 10⁻⁸ mol/l. En 26 h, les algues fixent 45% de la quantité d'uranium initialement présente en solution. Dans ces conditions, le facteur de bioconcentration de l'uranium par les algues *Coccomyxae actinabiotis* est de 1300 l/kg de masse fraîche, soit environ 13000 l/kg de masse sèche. Les facteurs de bioconcentration rapportés dans la littérature sont de l'ordre de 120-140 l/kg pour des algues d'eau douce ou marines (Paquet et al., Toxicologie nucléaire, environnementale et humaine, Lavoisier ed, 2009, Editions TEC et DOC. Chap. 23 Uranium, p. 411-443).

### EXEMPLE 6 : DÉCONTAMINATION D'UNE EAU DE PISCINE D'ENTREPOSAGE AU MOYEN DE COCCOMYXAE ACTINABIOTIS PAR ACTION IN SITU

Les micro-algues *Coccomyxae actinabiotis* ont été placées sur des supports horizontaux dans une piscine de refroidissement d'éléments combustibles d'une installation nucléaire. Cette piscine est remplie d'eau de pH moyen 5,5, de conductivité moyenne 1,2 µS/cm, de température moyenne 25°C, et contient des éléments métalliques radioactifs du fait des pièces qui y sont entreposées. La piscine est en contact avec l'air ambiant et est illuminée par des néons. L'intensité lumineuse à la surface de l'eau est de 200 lux. Selon les pièces entreposées dans la piscine, le débit de dose au niveau des algues varie d'une centaine de µGy/h à quelques dizaines de Gy/h. Dans ces conditions, *Coccomyxae actinabiotis* est capable de coloniser les supports sur lesquels elle est placée, et peut y vivre et s'y reproduire pendant des années. De manière ponctuelle, le débit de dose peut atteindre quelques centaines de Gy/h.

En effectuant des comptages par spectrométrie γ sur des échantillons de 50 ml d'eau et de 100 mg d'algues prélevées à partir des supports, l'activité totale ainsi que la nature et l'activité de chaque émetteur γ présent ont été déterminées. Les résultats obtenus après un temps de séjour de 5 ans sont illustrés par le Tableau IV ci-dessous.

**Tableau IV:**

| Radionucléide | Activité (Bq) de 1 ml d'eau | Activité (Bq) de 1 g de micro-algues |
|---|---|---|
| ⁵¹Cr | 1,73 | 10543 |
| ⁵⁴Mn | 0,15 | 168 |
| ⁶⁰Co | 0,64 | 24733 |
| ⁶⁵Zn | 0,13 | 363 |
| ^{108m}Ag | < limite de détection=0,02 | 556 |
| ^{110m}Ag | 0,006 | 2671 |
| ¹²⁴Sb | 0,87 | 149 |

Ces résultats montrent que les algues sont environ 10000 fois plus actives que l'eau dans laquelle elles vivent : les radioéléments se trouvent effectivement concentrés à l'extérieur et à l'intérieur des micro-algues.

L'argent en particulier a quasiment disparu de l'eau. Le cobalt et le chrome ont été très fortement concentrés.

### EXEMPLE 7 : DÉCONTAMINATION D'UNE EAU DE PISCINE D'ENTREPOSAGE AU MOYEN DE COCCOMYXAE ACTINABIOTIS PAR ACTION IN SITU ET COMPARAISON AUX MÉTHODES CLASSIQUES

Une expérience de décontamination d'une eau de piscine d'entreposage d'éléments radioactifs, de volume total 361 m³, a été réalisée in situ en utilisant la micro-algue *Coccomyxa actinabiotis* en suspension dans la piscine à dépolluer. A t = 14 jours, deux barres de sécurité issue d'un réacteur ont été introduites dans la piscine. Ces barres relarguent de l'argent 110m. L'eau est habituellement épurée par des résines échangeuses d'ions qui fixent les radionucléides. A t = 30 j, l'épuration par les résines a été stoppée. De t = 35 j à t = 56 j, l'eau a été épurée par captation des radionucléides par les algues en suspension dans la piscine. Ces algues, ainsi que l'activité radiologique collectée, viennent se fixer sur des filtres micro-pores (diamètre 50 mm, hauteur 60 mm) installés dans un robot de piscine mobile en surface. Les filtres, très actifs, sont changés tous les 2 jours.

La Figure 10 présente la concentration en argent 110m dans cette piscine en fonction du temps, exprimé en nombre de jours. La décroissance de la concentration en ^{110m}Ag observée entre 35 et 56 j est due à sa captation par les algues présentes dans la piscine et qui se fixent sur les filtres.

A partir de t = 60 j, les résines échangeuses d'ions ont été remises en fonctionnement. La Figure 10 montre une épuration des radioéléments avec une efficacité voisine qu'elle soit réalisée par les micro-algues ou par les résines échangeuses d'ions. Au total, durant cette expérience, 740 MBq d'émetteurs γ (dont 470 pour ^{110m}Ag, 180 pour ¹²⁴Sb et 90 pour ⁶⁰Co) ont été évacués de la piscine par les algues en 21 jours.

### EXEMPLE 8 : DÉCONTAMINATION D'EAUX D'INSTALLATIONS NUCLÉAIRES AU MOYEN DE COCCOMYXAE ACTINABIOTIS PAR MÉTHODE DÉPORTÉE

Une eau provenant d'une piscine de stockage d'éléments nucléaires usés a été mise en contact avec des *Coccomyxae actinabiotis* (50 ml d'eau en contact avec 9.10⁸ cellules soit 140 mg d'algues en masse fraîche provenant de culture et préalablement rincées 3 fois avec de l'eau milli-Q). L'eau contient initialement les radionucléides listés dans le Tableau V ci-dessous.

Le dosage de l'eau et des algues 24 h après la mise en contact montre une élimination de 92% de l'activité γ de l'eau en 24h.

Le Tableau V ci-dessous indique la composition initiale de l'eau, selon l'activité γ mesurée, totale et par radionucléide, et le pourcentage de radionucléides émetteurs γ fixés par les algues en 24 h.

**Tableau V :**

| | Total | ⁵⁴Mn | ⁶⁰Co | ^{110m}Ag | ¹³⁷Cs | ²³⁸U |
|---|---|---|---|---|---|---|
| Activité γ initiale dans l'eau (Bq/l) | 161,9 | 9,2 | 42,4 | 22,7 | 66,5 | 21 |
| % de fixation | 92 | 100 | 72 | 100 | 100 | 95 |

La fixation est donc de 100% pour l'argent 110m, le manganèse 54 et le césium 137, de 72 % pour le cobalt 60, et de 95 % pour l'uranium 238.

### EXEMPLE 9 : MAITRISE DE LA PROLIFERATION DES MICRO-ALGUES

Les micro-algues sont photosynthétiques. Elles ont besoin de lumière pour réaliser la photosynthèse et fabriquer leur matière organique. Dans le milieu très pauvre en nutriments qui correspond aux divers milieux et effluents des centrales nucléaires, leur croissance peut donc être maîtrisée par l'illumination. Pour qu'elles se développent à un endroit donné, il suffit de les éclairer. Dans la piscine où elles ont été découvertes, elles prolifèrent de préférence au voisinage des sources de lumière. Lorsqu'elles sont placées à l'obscurité pendant une durée de 2 jours à 1 mois, leur croissance cesse. Leur croissance peut aussi être maîtrisée en les éclairant avec une lumière qui ne permette pas ou peu de photosynthèse, par exemple avec une lampe inactinique de couleur jaune-verte.

Une filtration des eaux permet de capter les algues en suspension dans l'eau, et d'en maîtriser la croissance.

## Revendications

1. Algue verte unicellulaire du genre *Coccomyxa* **caractérisée en ce qu'**elle appartient à l'espèce *Coccomyxa actinabiotis* définie par la présence, dans les gènes d'ARN ribosomique 18S-ITS1-ARNr5.8S-ITS2-ARNr26S, d'une séquence présentant au moins 95% d'identité avec la séquence SEQ ID NO:

2. Souche de *Coccomyxa actinabiotis* déposée le 25 juin 2009 auprès de la CCAP sous le numéro CCAP 216/25.

3. Procédé de captage d'au moins un élément choisi parmi les métaux Ag, Co, Cr, Zn, Mn, Sb, Ni, Fe, Cs, actinides, et lanthanides, et les radioisotopes ¹⁴C et ³H, à partir d'un milieu aqueux contenant ledit métal ou ledit radioisotope en solution, **caractérisé en ce que** ledit captage est effectué en incubant les algues vertes unicellulaires du genre *Coccomyxa* telles que définies dans la revendication 1 ou dans la revendication 2 dans ledit milieu aqueux.

4. Procédé selon la revendication 3, **caractérisé en ce que** ledit milieu aqueux est un milieu radioactif.

5. Procédé selon la revendication 4 pour le captage d'un métal choisi parmi Ag, Co, Cr, Zn, Mn, Sb, Ni, Fe, Cs, actinides, et lanthanides, **caractérisé en ce que** ledit métal est sous forme d'isotope radioactif, ou sous forme de mélange d'isotopes.

6. Procédé selon l'une quelconque des revendications 3 à 5, **caractérisé en ce que** lesdites algues vertes sont associées à au moins un autre microorganisme et/ou au moins une plante multicellulaire.

7. Procédé selon l'une quelconque des revendications 3 à 6, **caractérisé en ce que** la croissance des algues vertes de l'espèce *Coccomyxa actinabiotis* telles que définies dans la revendication 1 ou dans la revendication 2 est maîtrisée en contrôlant l'illumination dudit milieu aqueux.

8. Procédé selon une quelconque des revendications 3 à 7, **caractérisé en ce que** l'élément capté est un métal, et **en ce qu'**il comprend une étape de récupération dudit métal à partir des algues.

9. Utilisation d'une algue verte du genre *Coccomyxa* telle que définie dans la revendication 1 ou dans la revendication 2 pour la décontamination d'un milieu aqueux radioactif contenant au moins un élément choisi parmi les métaux Ag, Co, Cr, Zn, Mn, Sb, Ni, Fe, Cs, actinides, et lanthanides, et les radioisotopes ¹⁴C et ³H.

10. Utilisation selon la revendication 9, **caractérisée en ce que** ladite algue verte est associé à au moins un autre microorganisme et/ou au moins une plante multicellulaire radiorésistant(e) ou radiotolérant(e).

## Patentansprüche

1. Einzellige Grünalge der Gattung *Coccomyxa,* **dadurch gekennzeichnet, dass** sie zu der Art *Coccomyxa actinabiotis* gehört, die durch das Vorliegen einer Sequenz, die wenigstens 95% Identität mit der Sequenz SEQ ID NO:1 aufweist, in den Genen von ribosomaler RNA 18S-ITS1-RNAr5.8S-ITS2-RNAr26S definiert ist.

2. Stamm von *Coccomyxa actinabiotis,* der am 25. Juni 2009 bei der CCAP unter der Nummer CCAP 216/25 hinterlegt wurde.

3. Verfahren zum Abfangen wenigstens eines Elementes, ausgewählt aus den Metallen Ag, Co, Cr, Zn, Mn, Sb, Ni, Fe, Cs, Actiniden und Lanthaniden und den Radioisotopen ¹⁴C und ³H, aus einem wässrigen Medium, welches das Metall oder das Radioisotop in Lösung enthält, **dadurch gekennzeichnet, dass** das Abfangen durchgeführt wird, indem die einzelligen Grünalgen der Gattung *Coccomyxa,* wie sie in Anspruch 1 oder Anspruch 2 definiert sind, in dem wässrigen Medium inkubiert werden.

4. Verfahren gemäß Anspruch 3, **dadurch gekennzeichnet, dass** das wässrige Medium ein radioaktives Medium ist.

5. Verfahren gemäß Anspruch 4 für das Abfangen eines Metalls, ausgewählt aus Ag, Co, Cr, Zn, Mn, Sb, Ni, Fe, Cs, Actiniden und Lanthaniden, **dadurch gekennzeichnet, dass** das Metall in Form eines radioaktiven Isotops oder in Form eines Isotopengemisches ist.

6. Verfahren gemäß einem der Ansprüche 3 bis 5, **dadurch gekennzeichnet, dass** die Grünalgen mit wenigstens einem anderen Mikroorganismus und/oder wenigstens einer mehrzelligen Pflanze assoziiert sind.

7. Verfahren gemäß einem der Ansprüche 3 bis 6, **dadurch gekennzeichnet, dass** das Wachstum der Grünalgen der Art *Coccomyxa actinabiotis,* wie sie in Anspruch 1 oder in Anspruch 2 definiert sind, reguliert wird, indem die Beleuchtung des wässrigen Mediums kontrolliert wird.

8. Verfahren gemäß einem der Ansprüche 3 bis 7, **dadurch gekennzeichnet, dass** das abgefangene Element ein Metall ist und dass es eine Stufe der Wiedergewinnung des Metalls aus den Algen umfasst.

9. Verwendung einer Grünalge der Gattung *Coccomyxa*, wie sie in Anspruch 1 oder in Anspruch 2 definiert ist, für die Dekontamination eines wässrigen radioaktiven Mediums, das wenigstens ein Element, ausgewählt aus den Metallen Ag, Co, Cr, Zn, Mn, Sb, Ni, Fe, Cs, Actiniden und Lanthaniden und den Radioisotopen ¹⁴C und ³H, enthält.

10. Verwendung gemäß Anspruch 9, **dadurch gekennzeichnet, dass** die Grünalge mit wenigstens einem anderen Mikroorganismus und/oder wenigstens einer mehrzelligen Pflanze, der/die gegenüber Radioaktivität resistent oder gegenüber Radioaktivität tolerant ist, assoziiert ist.

## Claims

1. A single-cell green alga of the *Coccomyxa* genus, **characterized in that** it belongs to the *Coccomyxa actinabiotis* species defined by the presence, in the 18S ribosomal RNA-ITS1-5.8S rRNA-ITS2-26S rRNA genes, of a sequence exhibiting at least 95% identity with the sequence SEQ ID NO: 1.

2. The *Coccomyxa actinabiotis* strain deposited on June 25, 2009 with the CCAP under number CCAP 216/25.

3. A method for taking up at least one element chosen from the metals Ag, Co, Cr, Zn, Mn, Sb, Ni, Fe, Cs, actinides and lanthanides, and the radioisotopes ¹⁴C and ³H, from an aqueous medium containing said metal or said radioisotope in solution, **characterized in that** said uptake is carried out by incubating single-cell green algae of the *Coccomyxa* genus as defined in claim 1 or in claim 2 in said aqueous medium.

4. The method as claimed in claim 3, **characterized in that** said aqueous medium is a radioactive medium.

5. The method as claimed in claim 4, for taking up a metal chosen from Ag, Co, Cr, Zn, Mn, Sb, Ni, Fe, Cs, actinides and lanthanides, **characterized in that** said metal is in the form of a radioactive isotope, or in the form of a mixture of isotopes.

6. The method as claimed in any one of claims 3 to 5, **characterized in that** said green algae are combined with at least one other microorganism and/or at least one multicellular plant.

7. The method as claimed in any one of claims 3 to 6, **characterized in that** the growth of the green algae of the *Coccomyxa actinabiotis* species as defined in claim 1 or in claim 2 is controlled by controlling the illumination of said aqueous medium.

8. The method as claimed in any one of claims 3 to 7, **characterized in that** the element taken up is a metal, and **in that** it comprises a step of recovering said metal from the algae.

9. The use of a green alga of the *Coccomyxa* genus as defined in claim 1 or in claim 2 for decontaminating a radioactive aqueous medium containing at least one element chosen from the metals Ag, Co, Cr, Zn, Mn, Sb, Ni, Fe, Cs, actinides and lanthanides, and the radioisotopes ¹⁴C and ³H.

10. The use as claimed in claim 9, **characterized in that** said green alga is combined with at least one other radioresistant or radiotolerant microorganism and/or at least one radioresistant or radiotolerant multicellular plant.
